# EUROPEAN PATENT APPLICATION

(11) **EP 1 258 254 A1**
(43) Date of publication of application: **20.11.2002**
(21) Application number: 01904560.8
(22) Date of filing: 20.02.2001
(51) Int. Cl.: A61K 47/30, A61K 9/14, A61K 9/19, A61K 31/4184

(54) **SUSTAINED RELEASE PREPARATIONS OF PHYSIOLOGICALLY ACTIVE COMPOUND HARDLY SOLUBLE IN WATER AND PRODUCTION PROCESS AND USE OF THE SAME**

(30) Priority: 21.02.2000 JP 2000048980
(71) Applicant: Takeda Chemical Industries, Ltd., Osaka-shi, Osaka 541-0045 (JP)
(72) Inventor: KAMEI, Shigeru, Takarazuka-shi, Hyogo 665-0847 (JP); OJIMA, Mami, Amagasaki-shi, Hyogo 661-0002 (JP); KITAYOSHI, Takahito, Suita-shi, Osaka 565-0821 (JP); IGARI, Yasutaka, Kobe-shi, Hyogo 658-0015 (JP)
(74) Representative: Teipel, Stephan
(86) International application number: JP0101191
(87) International publication number: WO01060410

(57) **Abstract**

A sustained-release preparation containing a physiologically active compound slightly soluble in water, a component obtained by treating with water a polyvalent metal compound slightly soluble in water, and a biodegradable polymer which are improved in the release-control and stabilization of the physiologically active compound slightly soluble in water and can be produced by a process suitable for mass production.

## Description

### Technical Field

The present invention relates to a sustained-release preparation of a physiologically active compound slightly soluble in water and its production process and use of the same.

### Background Art

Sustained-release preparations of a physiologically active compound containing a polyvalent metal slightly soluble in water and a biodegradable polymer are disclosed, for example, in JP 9-221420 A, JP 11 315034 A, JP 11-322631 A and the like and it is confirmed that release-control and stabilization of physiologically active compounds are possible. However, in the production of these sustained-release preparations, at the time of preparing an emulsion (S/O emulsion) containing a physiologically active compound slightly soluble in water, a polyvalent metal slightly soluble in water and a biodegradable polymer, for example, a stirring operation for several hours to half a day or longer is required.

On the other hand, a polyvalent metal compound slightly soluble in water is known to be almost insoluble in water and generally have an extremely slow bonding rate with water and, for example, Chemical Glossary describes that zinc oxide has solubility of 0.42 mg/100 ml (18°C), i.e., almost insoluble in water and that a conversion rate of zinc oxide into zinc hydroxide by bonding with water is extremely slow.

### Disclosure of the Invention

The present invention provides a sustained-release preparation with improved release-control and stabilization of a physiologically active compound which can be produced by a process suitable for mass production. A production process and use thereof are also provided.

In order to solve the above problem, the present inventors extensively studied, in particular, a process for producing a sustained-release preparation of a physiologically active compound, capable of preparing an emulsion containing a physiologically active compound slightly soluble in water, a polyvalent metal slightly soluble in water and a biodegradable polymer within a short time (preferably within one hour). As a result, the present inventors found that an emulsion containing a component obtained by treating zinc oxide with water, a physiologically active compound slightly soluble in water, and a biodegradable polymer can be prepared within a short time and further that a sustained-release preparation obtained by removing water and a solvent from the emulsion can perform sustained-release of the physiologically active compound contained in a high concentration with controlling the release. The present inventors made further investigations based on this finding, and completed the present invention.

That is, the present invention relates to:
(1) A sustained-release preparation which comprises a physiologically active compound slightly soluble in water, a component obtained by treating with water a polyvalent metal compound slightly soluble in water (e.g., a polyvalent metal compound slightly soluble in water treated by water which is obtained by mixing the polyvalent metal compound slightly soluble in water with water, and the like, in this case, a part or all of the polyvalent metal compound slightly soluble in water treated by water may be the polyvalent metal compound slightly soluble in water (unchanged)), and a biodegradable polymer;
(2) The sustained-release preparation according to the above (1), wherein the physiologically active compound is a non-peptide compound;
(3) The sustained-release preparation according to the above (1), wherein the physiologically active compound is a compound having angiotensin II antagonistic activity, its prodrug, or a salt thereof;
(4) The sustained-release preparation according to the above (3), wherein the compound having angiotensin II antagonistic activity is a non-peptide compound;
(5) The sustained-release preparation according to the above (3), wherein the compound having angiotensin II antagonistic activity is a compound containing oxygen atom in its molecule;
(6) The sustained-release preparation according to the above (3), wherein the compound having angiotensin II antagonistic activity is a compound having an ether bond or a carbonyl group;
(7) The sustained-release preparation according to the above (3), wherein the compound having angiotensin II antagonistic activity is a compound represented by the formula I: 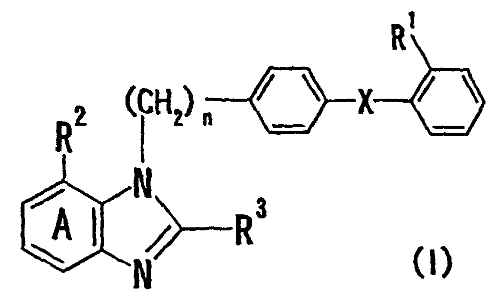 wherein R¹ is a group capable of forming an anion or a group capable of converting thereinto, X shows that the phenylene group and the phenyl group bind to each other directly or through a spacer having an atomic chain length of 2 or less, n is an integer of 1 or 2, the ring A is a benzene ring having an optional substitution, in addition to the group R², R² is a group capable of forming an anion or a group capable of converting thereinto, and R³ is an optionally substituted hydrocarbon residue which may bind through a hetero-atom, or a salt thereof;
(8) The sustained-release preparation according to the above (3), wherein the compound having angiotensin II antagonistic activity is Losartan, Eprosartan, Candesartan cilexetil, Candesartan, Valsartan, Telmisartan, Irbesartan, Ormesartan, or Tasosartan;
(9) The sustained-release preparation according to the above (3), wherein the compound having angiotensin II antagonistic activity is 2-ethoxy-1-[[2'-(1H-tetrazol-5-yl)biphenyl-4-yl]methyl]benzimidazole-7-carboxylic acid or a salt thereof;
(10) The sustained-release preparation according to the above (3), wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate or a salt thereof;
(11) The sustained-release preparation according to the above (3), wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid or a salt thereof;
(12) The sustained-release preparation according to the above (1), wherein the biodegradable polymer is α-hydroxycarboxylic acid polymer;
(13) The sustained-release preparation according to the above (12), wherein the α-hydroxycarboxylic acid polymer is lactic acid-glycolic acid polymer;
(14) The sustained-release preparation according to the above (13), wherein the molar ratio of lactic acid and glycolic acid is 100/0-40/60;
(15) The sustained-release preparation according to the above (12), wherein the weight-average molecular weight of the polymer is 3,000-50,000;
(16) The sustained-release preparation according to the above (1), which is for injection;
(17) The sustained-release preparation according to the above (1), wherein the polyvalent metal is zinc;
(18) The sustained-release preparation according to the above (17), wherein the polyvalent metal compound is zinc oxide;
(19) The sustained-release preparation according to the above (1) which further comprises a polyvalent metal;
(20) The sustained-release preparation according to the above (19), wherein the polyvalent metal is zinc;
(21) A process for producing the sustained-release preparation according to the above (1), which comprises removing water and a solvent from a solution containing a physiologically active compound slightly soluble in water, a component obtained by treating with water a polyvalent metal compound slightly soluble in water, and a biodegradable polymer;
(22) The process for producing the sustained-release preparation according to the above (21), wherein the physiologically active compound is a compound having angiotensin II antagonistic activity, its prodrug, or a salt thereof;
(23) The process for producing the sustained-release preparation according to the above (19), which comprises removing water and a solvent from a solution containing a physiologically active compound slightly soluble in water, a component obtained by treating with water a polyvalent metal compound slightly soluble in water, a biodegradable polymer, and a polyvalent metal;
(24) The process for producing the sustained-release preparation according to the above (23), wherein the physiologically active compound is a compound having angiotensin II antagonistic activity, its prodrug, or a salt thereof;
(25) A pharmaceutical composition comprising the sustained-release preparation according to the above (1);
(26) The composition according to the above (25) for a prophylactic and therapeutic drug for cardiovascular diseases;
(27) The composition according to the above (25) for a prophylactic and therapeutic drug for hypertension;
(28) The composition according to the above (25) for a prophylactic and therapeutic drug for blood pressure within-day precision abnormality;
(29) The composition according to the above (25) for a prophylactic and therapeutic drug for organ disorder;
(30) The sustained-release preparation according to the above (1), wherein the component obtained by treating with water a polyvalent metal compound slightly soluble in water is a component obtained by mixing the polyvalent metal compound slightly soluble in water with water;
(31) A process for producing a sustained-release preparation of a physiologically active compound slightly soluble in water, which comprises removing water and a solvent from an emulsion obtained by mixing the physiologically active compound slightly soluble in water, a polyvalent metal compound slightly soluble in water and water with a solution of a biodegradable polymer in an organic solvent;
(32) A process for producing a sustained-release preparation of a physiologically active compound slightly soluble in water, which comprises dispersing a physiologically active compound slightly soluble in water in an emulsion obtained by mixing a polyvalent metal compound slightly soluble in water and water with a solution of a biodegradable polymer in an organic solvent, and then removing water and a solvent from the dispersion;
(33) A sustained-release preparation obtained by the process according to the above (31);
(34) The sustained-release preparation according to the above (33), wherein water to be mixed is a release controlling agent for a physiologically active compound slightly soluble in water;
(35) A sustained-release preparation obtained by the process according to the above (32);
(36) The sustained-release preparation according to the above (35), wherein water to be mixed is a release controlling agent for a physiologically active compound slightly soluble in water;
(37) A method for controlling the release rate of a physiologically active compound slightly soluble in water from a sustained-release preparation, which comprises removing water and a solvent from a solution containing a physiologically active compound slightly soluble in water and a biodegradable polymer in the presence of a compound obtained by treating with water a polyvalent metal compound slightly soluble in water;
(38) The method according to the above (37), wherein the amount of water to be treated is controlled (increase, decrease, etc.);
(39) The method according to the above (37), wherein water and a solvent are removed from an emulsion obtained by mixing a physiologically active compound slightly soluble in water and a polyvalent metal compound slightly soluble in water with a solution of a biodegradable polymer in an organic solvent in the presence of water;
(40) The method according to the above (37), wherein a physiologically active compound slightly soluble in water is dispersed in an emulsion obtained by mixing a polyvalent metal compound slightly soluble in water with a solution of a biodegradable polymer in an organic solvent in the presence of water, and water and a solvent are removed from the dispersion;
(41) An emulsion comprising an internal phase containing a physiologically active compound slightly soluble in water, a polyvalent metal compound slightly soluble in water, and water in the internal phase, and an external phase containing a solution of a biodegradable polymer in an organic solvent; and the like.

In the present specification, the term "slightly soluble in water" means that the amount of water or a physiological saline solution required for dissolving a solute of 1 g within 30 minutes under conditions of strongly stirring for 30 seconds every 5 minutes at 20 ± 5°C is at least 100 ml or more, preferably 1,000 ml or more, more preferably 10,000 ml or more.

The term "physiologically active compound" in the present invention means that a physiologically active compound effective to prophylactic and therapeutics for diseases and illness of mammals (e.g., human being, cattle, pigs, dogs, cats, mice, rats, rabbits and the like). It is not particularly limited in so far as it has a sustained-release property or is stabilized owing to interaction with a component obtained by treatment with water a polyvalent metal compound slightly soluble in water and may be peptide type or non-peptide type, but non-peptide type ones are preferable. Among them, a physiologically active compound with a molecular weight of about 200 to 3,000 is preferable and a physiologically active compound with a molecular weight of about 300 to 2,000 is more preferable.

Preferable examples of "a physiologically active compound slightly soluble in water" to be employed in the present invention include a compound having angiotensin II antagonistic activity (a compound having angiotensin II receptor antagonistic activity), its prodrug and their salt.

In the present specification, the angiotensin II antagonistic activity is to inhibit competitively or non-competitively binding of angiotensin II to the angiotensin II receptors on the cellular membrane so as to reduce potent vasoconstrictive action or vascular smooth muscle proliferation action induced by angiotensin II and to ameliorate the symptom of hypertension.

The compound having angiotensin II antagonistic activity to be used for the present invention may be either a peptide compound or a non-peptide compound. In view of the advantage of long action, a non-peptide compound having angiotensin II antagonistic activity is preferable. As the compound having angiotensin II antagonistic activity, a compound having an oxygen atom in its molecule is preferable, a compound having an ether linkage or a carbonyl group (said carbonyl group may form a hydroxy group by resonance) is more preferable, a compound having an ether linkage or a ketone derivative is further preferable, and in particular, an ether derivative is preferable.

Any non-peptide compound having angiotensin II antagonistic activity can be used for the present invention. Examples of said compounds include imidazole derivatives disclosed in JP 56-71073 A, JP 56-71074 A, JP 57-98270 A, JP 58-157768 A, USP 4,355,040, USP 4,340,598, etc.; modified imidazole derivatives disclosed in EP-253310, EP-291969, EP-324377, EP-403158, WO-9100277, JP 63-23868 A, JP 1-117876 A, etc.; pyrrole, pyrazole and triazole derivatives disclosed in USP 5,183,899, EP-323841, EP-409332, JP 1-287071 A, etc.; benzimidazole derivatives disclosed in USP 4,880,804, EP-0392317, EP-0399732, EP-0400835, EP-425921, EP-459136, JP 3-63264 A, etc.; azaindene derivatives disclosed in EP-399731, etc.; pyrimidone derivatives disclosed in EP-407342, etc.; quinazoline derivatives disclosed in EP-411766, etc.; xanthine derivatives disclosed in EP-430300, etc.; fused imidazole derivatives disclosed in EP-434038, etc.; pyrimidinedione derivatives disclosed in EP-442473, etc.; thienopyridone derivatives disclosed in EP-443568, etc.; heterocyclic compounds disclosed in EP-445811, EP-483683, EP-518033, EP-520423, EP-588299, EP-603712, etc. In addition, their representative compounds are described in Journal of Medicinal Chemistry, Vol. 39, No. 3, pages 625-656 (1996). As the non-peptide compound having angiotensin II antagonistic activity, any one in addition to the compounds described in the above-mentioned references can be employed as far as it has angiotensin II antagonistic activity. Among others, Losartan (DuP753), Eprosartan (SK&F108566), Candesartan cilexetil (TCV-116), Valsartan (CGP-48933), Telmisartan (BIBR277), Irbesartan (SR47436), Tasosartan (ANA-756), their active metabolites (Candesartan, etc.), etc. are preferable.

Preferred examples of the non-peptide compound having angiotensin II antagonistic activity include, for example, a benzimidazole derivative of the formula (I): 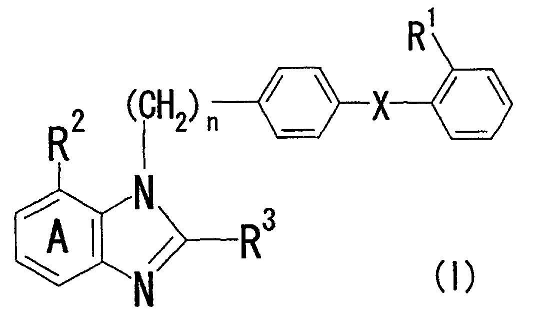 wherein R¹ is a group capable of forming an anion or a group capable of converting thereinto, X shows that the phenylene group and the phenyl group bind to each other directly or through a spacer having an atomic chain length of 2 or less, n is an integer of 1 or 2, the ring A is a benzene ring having an optional substitution, in addition to the group R², R² is a group capable of forming an anion or a group capable of converting thereinto, and R³ is an optionally substituted hydrocarbon residue which may bind through a hetero-atom (preferably, an optionally substituted hydrocarbon residue which binds through an oxygen atom), etc., or a salt thereof.

In the above formula (I), the group capable of forming an anion (a group having a hydrogen atom capable of leaving as a proton) as R¹ include, for example, (1) a carboxyl group, (2) a tetrazolyl group, (3) a trifluoromethanesulfonic acid amido group (-NHSO₂CF₃), (4) a phosphono group, (5) a sulfo group, (6) an optionally substituted 5- to 7-membered (preferably 5- to 6-membered) monocyclic heterocyclic ring residue which contains one or more of N, S and O, etc.

Examples of the above "optionally substituted 5- to 7-membered (preferably 5- to 6-membered) monocyclic heterocyclic ring residue which contains one or more of N, S and O" include 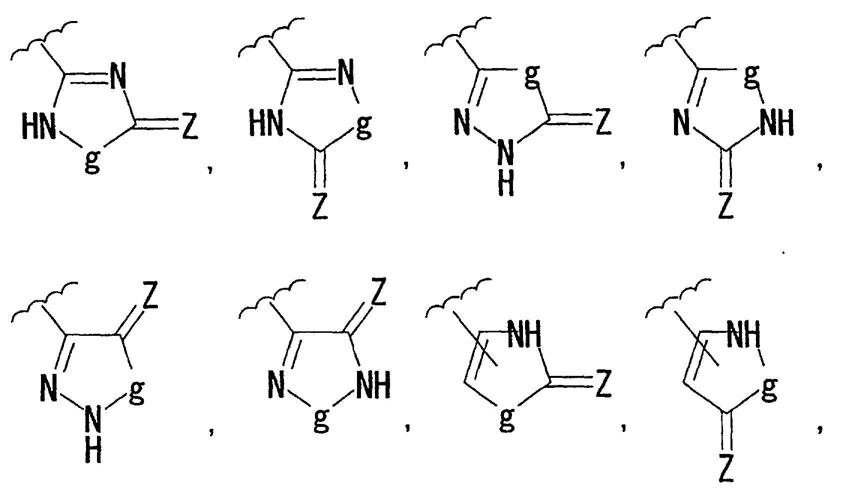 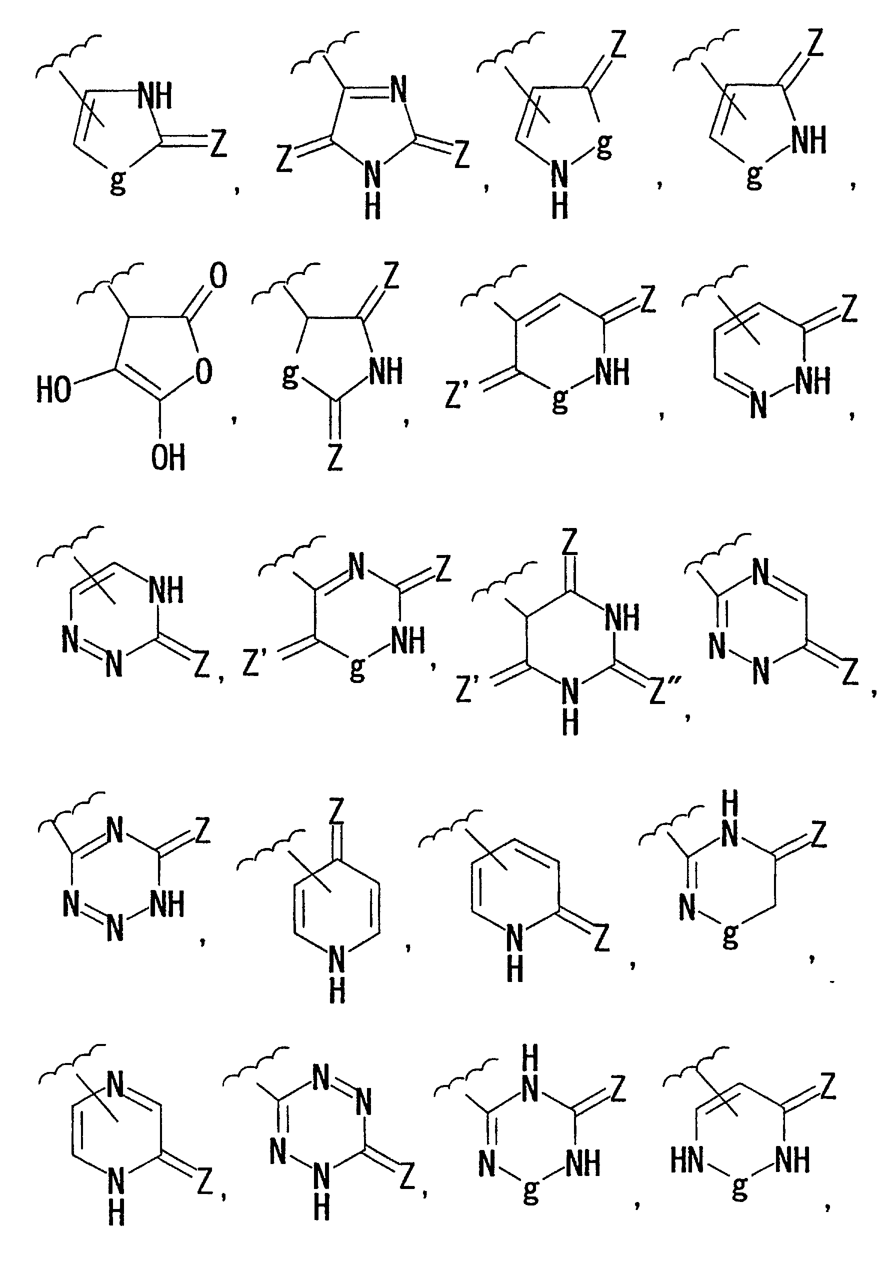 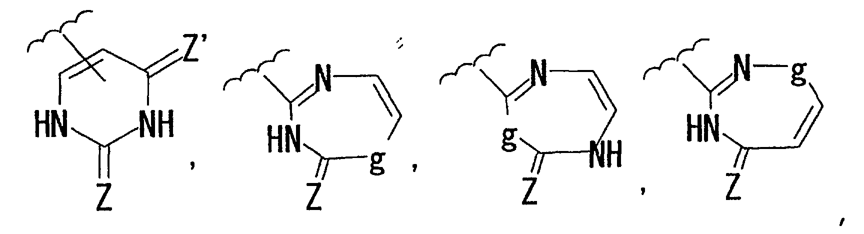 etc. The chemical bond between the heterocyclic ring residue represented by R¹ and the phenyl group to which said heterocyclic ring residue binds may be a carbon-carbon bond as shown above, or a nitrogen-carbon bond via one of the several nitrogen atoms when the symbol g is -NH-, etc. in the above formulas.

For example, when R¹ is represented by the formula: 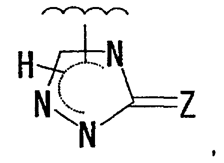 its specific embodiments are 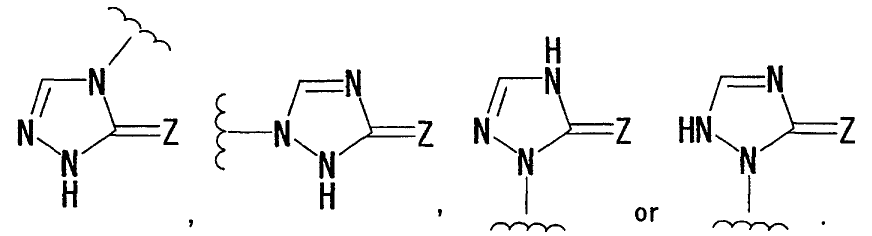 Other examples of R¹ binding through a nitrogen atom include

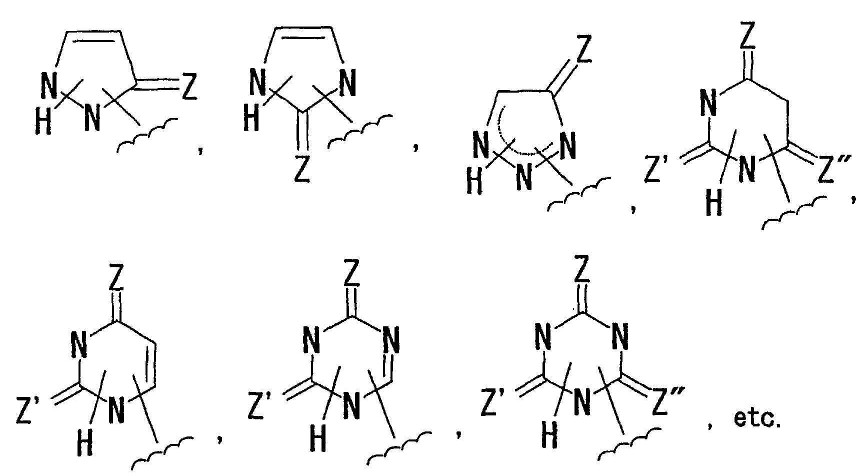

In the above formula, g is -CH₂-, -NH-, -O- or -S(O)m-; >=Z, >=Z' and >=Z'' are independently a carbonyl group, a thiocarbonyl group or an optionally oxidized sulfur atom (e.g., S, S(O), S(O)₂, etc.) (preferably a carbonyl group or a thiocarbonyl group, more preferably carbonyl group); and m is an integer of 0, 1 or 2.

Preferred examples of the heterocyclic ring residue represented by R¹ include a heterocyclic ring residue simultaneously having -NH- or -OH group as proton donor and a carbonyl group, a thiocarbonyl group, a sulfinyl group, etc. as proton acceptor, such as an oxadiazolone ring, an oxadiazolothione ring or an thiadiazolone ring, etc.

While the heterocyclic ring residue represented by R¹ may form a condensed ring by connecting the substituents on the heterocyclic ring, it is preferably 5- to 6-membered ring residue, more preferably 5-membered ring residue.

Preferred examples of the heterocyclic ring residue represented by R¹ include a group of the formula: 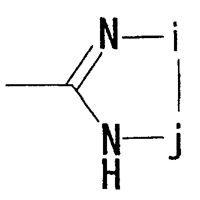 wherein i is -O- or -S-, j is >=O, >=S or >=S(O)m,
and m is as defined above (preferably, 4,5-dihydro-5-oxo-1,2,4-oxadiazol-3-yl, 4,5-dihydro-5-thioxo-1,2,4-oxadiazol-3-yl, 4,5-dihydro-5-oxo-1,2,4-thiadiazol-3-yl; more preferably, 4,5-dihydro-5-oxo-1,2,4-oxadiazol-3-yl).

The above-mentioned heterocyclic ring residue (R¹) may have the following tautomeric isomers. For example, in 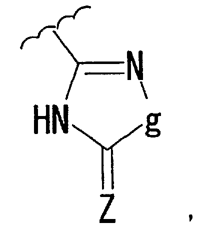 when Z is 0 and g is 0, 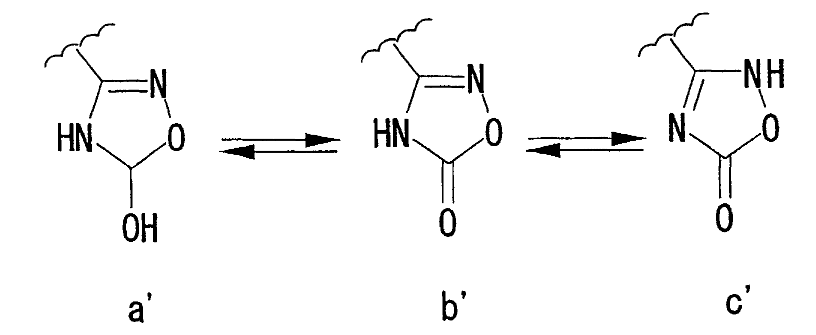 the three tautomeric isomers a', b' and c' exist and a group of the formula: 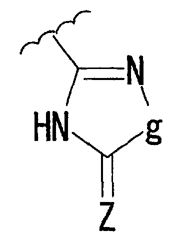 include all of the above a', b' and c'.

The group capable of forming an anion as R¹ may be protected by an optionally substituted lower (C₁₋₄) alkyl group, an acyl group (e.g., lower (C₂₋₅) alkanoyl, benzoyl, etc.), etc. at its possible position.

Examples of the optionally substituted lower (C₁₋₄) alkyl group include (1) a lower (C₁₋₄) alkyl group optionally substituted with one to three phenyl groups which may have halogen atom, nitro, lower (C₁₋₄) alkyl, lower (C₁₋₄) alkoxy, etc. (e.g., methyl, triphenylmethyl, p-methoxybenzyl, p-nitrobenzyl, etc.); (2) a lower (C₁₋₄) alkoxy-lower (C₁₋₄) alkyl group (e.g., methoxymethyl, ethoxymethyl, etc.); (3) a group of the formula: -CH(R⁴)-OCOR⁵ wherein R⁴ is (a) a hydrogen, (b) a straight or branched lower C₁₋₆ alkyl group (e.g., methyl, ethyl, n-propyl, isopropyl, n-butyl, isobutyl, t-butyl, n-pentyl, isopentyl, neopentyl, etc.), (c) a straight or branched lower C₂₋₆ alkenyl group or (d) a C₃₋₈ cycloalkyl group (e.g., cyclopentyl, cyclohexyl, cycloheptyl, etc.), and R⁵ is (a) a straight or branched lower C₁₋₆ alkyl group (e.g., methyl, ethyl, n-propyl, isopropyl, n-butyl, isobutyl, sec-butyl, t-butyl, n-pentyl, isopentyl, neopentyl, etc.), (b) a straight or branched lower C₂₋₆ alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., a phenyl group or a naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, a nitro, a lower (C₁₋₄) alkyl, a lower (C₁₋₄) alkoxy, etc.) such as phenoxy, p-nitrophenoxy, naphthoxy, etc.; etc.

The group capable of forming an anion as R¹ may be substituted, in addition to the above protective group such as an optionally substituted lower (C₁₋₄) alkyl group or an acyl group (e.g., lower (C₂₋₅) alkanoyl, benzoyl, etc.), etc., with an optionally substituted lower (C₁₋₄) alkyl group (e.g. an optionally substituted lower (C₁₋₄) alkyl group similar to the "optionally substituted lower (C₁₋₄) alkyl group" exemplified as a protective group for the above group capable of forming an anion as R¹), a halogen atom, a nitro, a cyano, a lower (C₁₋₄) alkoxy, an amino optionally substituted with 1 to 2 lower (C₁₋₄) alkyl groups, etc., at the possible position.

In the above formula, the group convertible into the group capable of forming an anion (a group having a hydrogen atom capable of leaving as proton) as R¹ may be a group convertible into a group capable of forming an anion under biological or physiological conditions (for example, in vivo reaction, etc. such as oxidation, reduction, hydrolysis, etc. by in vivo enzyme, etc.) [so called prodrug], or the group convertible into a group capable of forming an anion represented by R¹ may be a group chemically convertible into a group capable of forming an anion, such as cyano, N-hydroxycarbamimidoyl group (-C(=N-OH)-NH₂), a group selected from the class consisting of (1) a carboxyl group, (2) a tetrazolyl group, (3) a trifluoromethanesulfonic acid amido group (-NHSO₂CF₃), (4) a phosphono group, (5) a sulfo group and (6) an optionally substituted monocyclic 5- to 7-membered (preferably 5- to 6-membered) monocyclic heterocyclic ring residue which contains one or more of N, S and O, each of which is protected with an optionally substituted lower (C₁₋₄) alkyl group or an acyl group, etc. [so called synthetic intermediate].

As the group R¹, (1) carboxyl, tetrazolyl or 4,5-dihydro-5-oxo-1,2,4-oxadiazol-3-yl (preferably, tetrazolyl), each of which may be protected with an optionally substituted lower (C₁₋₄) alkyl (e.g., methyl, triphenylmethyl, methoxymethyl, ethoxymethyl, p-methoxybenzyl, p-nitrobenzyl, etc.) or an acyl group (e.g., lower (C₂₋₅) alkanoyl, benzoyl, etc.) or (2) cyano or N-hydroxycarbamimidoyl (preferably cyano) is preferable.

In the above formula, X shows that the phenylene group is bonded to the adjacent phenyl group directly or through a spacer with an atomic chain of 2 or less (preferably directly). Examples of the spacer with an atomic chain of 2 or less include any divalent chain in which the number of atoms constituting the straight chain is 1 or 2 and which may have a side chain, and specifically lower (C₁₋₄) alkylene in which the number of atoms constituting the straight chain is 1 or 2, -CO-, -O-, -S-, -NH-, -CO-NH-, -O-CH₂-, -S-CH₂-, -CH=CH-, etc.

In the above formula, n is an integer of 1 or 2 (preferably 1).

In the above formula, the ring A may have, in addition to the group R², another substituent, for example, (1) halogen (e.g., F, Cl, Br, etc.), (2) cyano, (3) nitro, (4) an optionally substituted lower (C₁₋₄) alkyl, (5) a lower (C₁₋₄) alkoxy, (6) an optionally substituted amino group (e.g., amino, N-lower (C₁₋₄) alkylamino (e.g., methylamino, etc.), N,N-di-lower (C₁₋₄) alkylamino (e.g., dimethylamino, etc.), N-arylamino (e.g., phenylamino, etc.), alicyclic amino (e.g., morpholino, piperidino, piperazino, N-phenylpiperazino, etc.), etc.), (7) a group of the formula: -CO-D' wherein D' is a hydroxy group or a lower (C₁₋₄) alkoxy whose alkyl moiety may be substituted with a hydroxy group, a lower (C₁₋₄) alkoxy, a lower (C₂₋₆) alkanoyloxy (e.g., acetoxy, pivaloyloxy, etc.), a lower (C₁₋₆) alkoxycarbonyloxy (e.g., methoxycarbonyloxy, ethoxycarbonyloxy, etc.) or a lower (C₃₋₆) cycloalkoxycarbonyloxy (e.g., cyclohexyloxycarbonyloxy, etc.), or (8) tetrazolyl, trifluoromethanesulfonic acid amide group, phosphono group or sulfo group, each of which may be protected with an optionally substituted lower (C₁₋₄) alkyl ("an optionally substituted lower (C₁₋₄) alkyl group" similar to that exemplified as a protective group for the above group capable of forming an anion represented by R¹, etc.) or acyl (e.g., lower (C₂₋₅) alkanoyl, benzoyl, etc.), etc.

Of these substituents, one or two may simultaneously be present at any possible position on the benzene ring, in addition to the group R², and preferred examples of the substituents for the benzene ring represented by A include an optionally substituted lower (C₁₋₄) alkyl (e.g., a lower (C₁₋₄) alkyl, etc. optionally substituted with a hydroxy group, a carboxyl group, a halogen, etc.), a halogen, etc. As the ring A, a benzene ring having no substituent in addition to the group R² is preferable.

In the above formula, examples of the group capable of forming an anion (a group having a hydrogen atom capable of leaving as proton) as R² include (1) an optionally esterified or amidated carboxyl group, (2) a tetrazolyl group, (3) a trifluoromethanesulfonic acid amido group (-NHSO₂CF₃), (4) a phosphono group, (5) a sulfo group, etc., each of which may be protected with an optionally substituted lower alkyl group (e.g. an optionally substituted lower (C₁₋₄) alkyl group similar to the "optionally substituted lower (C₁₋₄) alkyl group" exemplified as a protective group for the above group capable of forming an anion as R¹) or an acyl group (e.g., lower (C₂₋₅) alkanoyl, benzoyl, etc.), or any one of the groups capable of converting thereinto under biological or physiological conditions (for example, in vivo reaction, etc. such as oxidation, reduction, hydrolysis, etc. by in vivo enzyme, etc.), or chemically.

Examples of the optionally esterified or amidated carboxyl as R² include a group of the formula: -CO-D wherein D is (1) a hydroxy group, (2) an optionally substituted amino (for example, amino, N-lower (C₁₋₄) alkylamino, N,N-di-lower (C₁₋₄) alkylamino, etc.) or (3) an optionally substituted alkoxy [e.g., (i) a lower (C₁₋₆) alkoxy group whose alkyl moiety is optionally substituted with a hydroxy group, an optionally substituted amino (e.g., amino, N-lower (C₁₋₄) alkylamino, N,N-di-lower (C₁₋₄) alkylamino, piperidino, morpholino, etc.), a halogen, a lower (C₁₋₆) alkoxy, a lower (C₁₋₆) alkylthio, a lower (C₃₋₈) cycloalkoxy or an optionally substituted dioxolenyl (e.g., 5-methyl-2-oxo-1,3-dioxolen-4-yl, etc.), or (ii) a group of the formula: -O-CH(R⁶)-OCOR⁷ wherein R⁶ is (a) a hydrogen, (b) a straight or branched C₁₋₆ lower alkyl group (e.g., methyl, ethyl, n-propyl, isopropyl, n-butyl, isobutyl, t-butyl, n-pentyl, isopentyl, neopentyl, etc.), (c) a straight or branched C₂₋₆ lower alkenyl group or (d) a C₃₋₈ cycloalkyl group (e.g., cyclopentyl, cyclohexyl, cycloheptyl, etc.), and R⁷ is (a) a straight or branched C₁₋₆ lower alkyl group (e.g., methyl, ethyl, n-propyl, isopropyl, n-butyl, isobutyl, sec-butyl, t-butyl, n-pentyl, isopentyl, neopentyl, etc.), (b) a straight or branched C₂₋₆ lower alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., a phenyl group or a naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, a nitro, a lower (C₁₋₄) alkyl, a lower (C₁₋₄) alkoxy, etc.) such as phenoxy, p-nitrophenoxy, naphthoxy, etc.], etc.

As R², an optionally esterified carboxyl is preferable, and its specific examples include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyryloxymethoxycarbonyl, isobutyryloxymethoxycarbonyl, 1-(ethoxycarbonyloxy)ethoxycarbonyl, 1-(acetoxy)-ethoxycarbonyl, 1-(isobutyryloxy) ethoxycarbonyl, cyclohexylcarbonyloxymethoxycarbonyl, benzoyloxy-methoxycarbonyl, cinnamyloxycarbonyl, cyclopentyl-carbonyloxymethoxycarbonyl, etc. The group R² may be any one of the groups capable of forming an anion under biological or physiological conditions (for example, in vivo reaction, etc. such as oxidation, reduction, hydrolysis, etc. by in vivo enzyme, etc.), the groups capable of chemically forming an anion (e.g., COO⁻, its derivative, etc.) or the groups capable of converting thereinto. The group R² may be a carboxyl group or its prodrug.

Preferred examples of the group R² include a group of the formula: -CO-D wherein D is (1) a hydroxy group or (2) a lower (C₁₋₄) alkoxy whose alkyl moiety is optionally substituted with a hydroxy group, an amino, a halogen, a lower (C₂₋₆) alkanoyloxy (e.g., acetoxy, pivaloyloxy , etc.), a lower (C₃₋₈) cycloalkanoyloxy, a lower (C₁₋₆) alkoxycarbonyloxy (e.g., methoxycarbonyloxy, ethoxycarbonyloxy, etc.), a lower (C₃₋₈) cycloalkoxycarbonyloxy (e.g., cyclohexyloxycarbonyloxy, etc.), a lower (C₁₋₄) alkoxy or a lower (C₃₋₈) cycloalkoxy. Among others, an esterified carboxyl with a lower (C₁₋₄) alkyl (preferably, methyl or ethyl) is preferable.

In the above formula, examples of the "hydrocarbon residue" in the "optionally substituted hydrocarbon residue which may bind through a hetero-atom" represented by R³ include (1) an alkyl group, (2) an alkenyl group, (3) an alkynyl group, (4) an cycloalkyl group, (5) an aryl group, (6) an aralkyl group, etc. Among others, an alkyl group, an alkenyl group and a cycloalkyl group are preferable.

Examples of the alkyl group of the above mentioned (1) include straight or branched lower alkyl group having about 1-8 carbon atoms such as methyl, ethyl, propyl, isopropyl, butyl, isobutyl, sec-butyl, t-butyl, pentyl, i-pentyl, hexyl, heptyl, octyl, etc.

Examples of the alkenyl group of the above mentioned (2) include straight or branched lower alkenyl group having about 2-8 carbon atoms such as vinyl, propenyl, 2-butenyl, 3-butenyl, isobutenyl, 2-octenyl, etc.

Examples of the alkynyl group of the above mentioned (3) include straight or branched lower alkynyl group having about 2-8 carbon atoms such as ethynyl, 2-propynyl, 2-butynyl, 2-pantynyl, 2-octynyl, etc.

Examples of the cycloalkyl group of the above (4) include a lower cycloalkyl having about 3-6 carbon atoms, such as cyclopropyl, cyclobutyl, cyclopentyl, cyclohexyl, etc.

Each of the above-mentioned alkyl group, alkenyl group, alkenyl group and cycloalkyl group may be substituted with hydroxy group, an optionally substituted amino group (e.g., amino, N-lower (C₁₋₄) alkylamino, N,N-di-lower (C₁₋₄) alkylamino, etc.), halogen , lower (C₁₋₄) alkoxy group, lower (C₁₋₄) alkylthio group, etc.

Examples of the aralkyl group of the above (5) include a phenyl-lower (C₁₋₄) alkyl, etc., such as benzyl, phenethyl, etc.

Examples of the aryl group of the above (6) include phenyl, etc.

Each of the above-mentioned aralkyl group and aryl group may be substituted, at any possible position on the benzene ring, with a halogen (e.g., F, Cl, Br, etc.), a nitro, an optionally substituted amino group (e.g., amino, N-lower (C₁₋₄) alkylamino, N,N-di-lower (C₁₋₄) alkylamino, etc.), a lower (C₁₋₄) alkoxy (e.g., methoxy, ethoxy, etc.), a lower (C₁₋₄) alkylthio (e.g., methylthio, ethylthio, etc.), a lower (C₁₋₄) alkyl (e.g., methyl, ethyl, etc.), etc.

Preferred examples of the "optionally substituted hydrocarbon residue" in the "optionally substituted hydrocarbon residue which may bind through a hetero-atom" represented by R³ include an optionally substituted alkyl or alkenyl group (e.g., a lower (C₁₋₅) alkyl or a lower (C₂₋₅) alkenyl group, each of which may be substituted with a hydroxy group, an amino group, a halogen, a lower (C₁₋₄) alkoxy group, etc.). Among others, a lower (C₁₋₅) alkyl (more preferably, ethyl) is preferable.

Preferred examples of the "hetero-atom" in the "optionally substituted hydrocarbon residue which may bind through a hetero-atom" represented by R³ include -O-, -S(O)m- [m is an integer of 0-2], -NR'- [R' is a hydrogen atom or a lower (C₁₋₄) alkyl], etc. Among others, -O- is preferable.

Among others, as R³, a lower (C₁₋₅) alkyl or a lower (C₂₋₅) alkenyl group, each of which may be substituted with a substituent selected from the class consisting of a hydroxy group, an amino group, a halogen and a lower (C₁₋₄) alkoxy group and which may bind through -O-, -S(O)m- [m is an integer of 0-2] or -NR'- [R' is a hydrogen atom or a lower (C₁₋₄) alkyl], etc. is preferable and a lower (C₁₋₅) alkyl or lower (C₁₋₅) alkoxy (in particular, ethoxy) is more preferable.

Among the non-peptide compounds having angiotensin II antagonistic activity and represented by the formula (I), a benzimidazole-7-carboxylic acid derivative of the formula (I'): 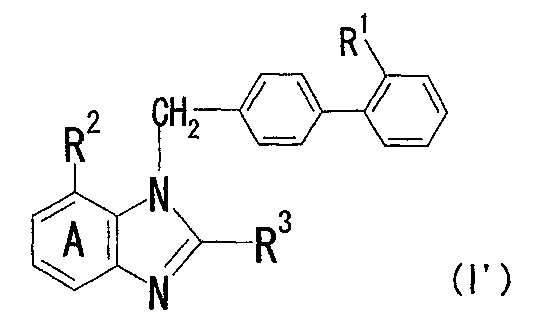 wherein R¹ is (1) carboxyl group, (2) tetrazolyl group or (3) a group of the formula: 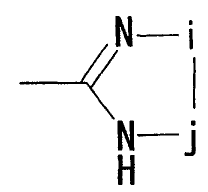 wherein i is -O- or -S-, j is >=O, >=S or >=S(O)m, and m is as defined above; the ring A is a benzene ring having an optional substituent selected from the class consisting of an optionally substituted lower (C₁₋₄) alkyl (e.g., a lower (C₁₋₄) alkyl optionally substituted with a hydroxy group, a carboxyl group, a halogen, etc.) and a halogen, in addition to the group R² (preferably, a benzene ring having no substituent in addition to the group R²); R² is a group of the formula: -CO-D wherein D is (1) a hydroxy group or (2) a lower (C₁₋₄) alkoxy whose alkyl moiety may be substituted with a hydroxy group, an amino, a halogen, a lower (C₂₋₆) alkanoyloxy (e.g., acetoxy, pivaloyloxy, etc.), a lower (C₃₋₈) cycloalkanoyloxy, a lower (C₁₋₆) alkoxycarbonyloxy (e.g., methoxycarbonyloxy, ethoxy-carbonyloxy, etc.), a lower (C₃₋₈) cycloalkoxycarbonyloxy (e.g., cyclohexyloxycarbonyloxy, etc.), a lower (C₁₋₄) alkoxy or a lower (C₃₋₈) cycloalkoxy; R³ is a lower (C₁₋₅) alkyl or a lower (C₂₋₅) alkenyl group, each of which may bind through -O-, -S(O)m- [m is an integer of 0-2] or -NR'- [R' is a hydrogen atom or a lower (C₁₋₄) alkyl] and which may be substituted with a substituent selected from the class consisting of a hydroxy group, an amino group, a halogen and a lower (C₁₋₄) alkoxy group (preferably, a lower (C₁₋₅) alkyl or a lower (C₁₋₅) alkoxy; more preferably, ethoxy), etc. or a pharmaceutically acceptable salt thereof is preferable.

Among others, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]-benzimidazole-7-carboxylate [Candesartan cilexetil], pivaloyloxymethyl 2-ethoxy-1-[[2'-(1H-tetrazol-5-yl)biphenyl-4-yl]methyl]benzimidazole-7-carboxylate, 2-ethoxy-1-[[2'-(4,5-dihydro-5-oxo-1,2,4-oxadiazol-3-yl)biphenyl-4-yl]methyl]benzimidazole-7-carboxylic acid, etc. or a salt thereof are preferable.

The above mentioned benzimidazole derivative can be produced by known methods described in, for example, EP-425921, EP-459136, EP-553879, EP-578125, EP-520423, EP-668272, etc. or a method analogous thereto. When Candesartan cilexetil is used for the present invention, a stable C-type crystal described in EP-459136 is preferably used.

The compound having angiotensin II antagonistic activity or a prodrug thereof may be distinct entity or in the form of any possible pharmaceutically acceptable salts thereof. Examples of said salts include a salt with inorganic bases (e.g., alkaline metals such as sodium, potassium, etc.; alkaline earth metals such as calcium, magnesium, etc.; transition metal such as zinc, iron, copper, etc.; etc.); organic bases (e.g., organic amines such as trimethylamine, triethylamine, pyridine, picoline, ethanolamine, diethanolamine, triethanolamine, dicyclohexylamine, N,N'-dibenzylethylenediamine, etc.; basic amino acids such as arginine, lysine, ornithine, etc.; etc.); etc., when said compound having angiotensin II antagonistic activity has an acidic group such as a carboxyl group, etc.; and a salt with inorganic acids or organic acids (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.); acidic amino acids such as aspartic acid, glutamic acid, etc.; etc., when said compound having angiotensin II antagonistic activity has a basic group such as an amino group, etc.

The prodrug of the compound having angiotensin II antagonistic activity [hereinafter, referred to as All antagonist] means a compound which is converted to All antagonist under the physiological condition or with a reaction due to an enzyme, an gastric acid, etc. in the living body, that is, a compound which is converted to All antagonist with oxidation, reduction, hydrolysis, etc. according to an enzyme; a compound which is converted to All antagonist with gastric acid, etc.; etc.

Examples of the prodrug of the All antagonist include a compound wherein an amino group of the All antagonist is substituted with acyl, alkyl, phosphoric acid, etc. (e.g. a compound wherein an amino group of the AII antagonist is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); a compound wherein an hydroxy group of the All antagonist is substituted with acyl, alkyl, phosphoric acid, boric acid, etc. (e.g. a compound wherein an hydroxy group of the AII antagonist is substituted with acetyl, palmitoyl, propanoyl, pivaloyl, succinyl, fumaryl, alanyl, dimethylaminomethylcarbonyl, etc.); a compound wherein a carboxyl group of the All antagonist is modified with ester, amide, etc. (e.g. a compound wherein a carboxyl group of the All antagonist is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These prodrugs can be produced by per se known method from the All antagonist.

The prodrug of the AII antagonist may be a compound which is converted into the All antagonist under the physiological conditions as described in "Pharmaceutical Research and Development", Vol. 7 (Drug Design), pages 163-198 published in 1990 by Hirokawa Publishing Co. (Tokyo, Japan).

The All antagonist may be either of hydrates or non-hydrates.

The term "a component obtained by treating with water a polyvalent metal compound slightly soluble in water" in the present invention means a component produced by treatment with water in the presence a polyvalent metal compound slightly soluble in water alone, or in the co-presence of a polyvalent metal compound slightly soluble in water and a physiologically active compound slightly soluble in water and/or a biodegradable polymer. The release control and stabilization of the physiologically active compound slightly soluble in water can easily achieved by changing treatment conditions, environments, and the like.

This treatment with water is done within a short period of time, for example, within 1 hour, preferably within 30 minutes, more preferably 10 minutes, furthermore preferably 5 minutes, and especially preferably 3 minutes.

The amount of water to be added differs depending on the amount of a polyvalent metal compound, the type and amount of a physiologically active compound, the type and amount of a biodegradable polymer, and the type and amount of a solution of the biodegradable polymer in an organic solvent and is generally 3 to 500%, preferably 5 to 300%, and more preferably 10 to 150% based on the weight of the polyvalent metal compound.

Water to be added may partially not be dissolved in an organic solvent for a biodegradable polymer and is preferably finely dispersed or emulsified by a known method using a homogenizer, ultrasonication, or the like.

If necessary, to water to be added, the following material(s) may be added: a polyvalent metal compound (e.g. zinc acetate and the like), a basic amino acid (e.g. arginine, histidine, lysine and the like), albumin, gelatin, agar, dextrin, polyvinyl alcohol, carbonic acid, oxalic acid, citric acid, phosphoric acid, hydrochloric acid, sodium hydroxide, citric acid, sodium ethylenediamine tetraacetate, sodium hydrogen sulfite, polyol compounds (e.g. polyethylene glycol), p-hydroxybenzoates (methylparaben, propylparaben and the like), benzylalcohol, chlorobutanol, thimerosal and the like.

Examples of the component obtained by treating a polyvalent metal compound slightly soluble in water with water include that obtained by, for example, mixing a polyvalent metal compound slightly soluble in water with water and, in this case, a part or all of the polyvalent metal compound slightly soluble in water treated with water may be the polyvalent metal compound slightly soluble in water (unchanged body). Further, in a step for producing the sustained-release preparation of the present invention, a polyvalent metal compound slightly soluble in water treated with water preferably forms an emulsion with a solution of a biodegradable polymer in an organic solvent in the co-presence of water. And, the sustained-release preparation is preferably produced via an emulsion composed of an inner phase containing a physiologically active compound slightly soluble in water, a polyvalent metal compound slightly soluble in water and water and an external phase containing a solution of a biodegradable polymer in an organic solvent, while it is possible to prepare a stable and uniform emulsion within a short period of time by forming the emulsion in the co-presence of water.

Examples of the biodegradable polymer to be used in the present invention include a polymer, a copolymer, their ester, or a mixture thereof which is synthesized from one or more of α-hydroxycarboxylic acids (e.g., glycolic acid, lactic acid, etc.), hydroxydicarboxylic acids (e.g., malic acid, etc.), hydroxytricarboxylic acids (e.g., citric acid, etc.), etc. and which has a free carboxyl group; poly-α-cyanoacrylic acid esters; polyamino acids (e.g., poly-g-benzyl-L-glutamic acid, etc.); maleic anhydride copolymer (e.g., styrene-maleic acid copolymer, etc.), etc.

The copolymers may be any of random, block and graft copolymers. When the above α-hydroxycarboxylic acids, hydroxydicarboxylic acids and hydroxytricarboxylic acids have optical activity in their molecules, any one of D-isomers, L-isomers and DL-isomers may be used. Among others, α-hydroxycarboxylic acid polymer (preferably lactic acid-glycolic acid polymer), its ester, poly-α-cyanoacrylic acid esters, etc. are preferable, and lactic acid-glycolic acid polymer, its ester are more preferable.

When the lactic acid-glycolic acid polymer is used as the biodegradable polymer, the molar ratio (mole %) ranges preferably from 100/0 to 40/60 and more preferably from 100/0 to 50/50.

In general, the weight-average molecular weight of the above lactic acid-glycolic acid polymer ranges from about 3,000 to about 50,000, preferably about 4,000 to about 40,000, and more preferably about 5,000 to about 30,000. Degree of dispersion (weight-average molecular weight/number-average molecular weight, hereinafter also referred to as dispersity) usually ranges from about 1.2 to about 4.0, preferably from about 1.5 to about 3.5.

In the present specification, the weight-average molecular weight, number-average molecular weight and dispersity mean molecular weights and dispersity determined by gel permeation chromatography (GPC) with 14 polymers of polystyrene as reference substances with weight-average molecular weights of 1,110,000, 707,000, 354,000, 189,000, 156,000, 98,900, 66,437, 37,200, 17,100, 9,830, 5,870, 2,500, 1,303 and 500, respectively. The determination was carried out using GPC column KF804L × 2 (manufactured by Showa Denko K.K., Japan) and using chloroform as the mobile phase. To calculate number-average molecular weight, the biodegradable polymer is dissolved in a mixed solvent of acetone and methanol and the solution is titrated with alcoholic potassium hydroxide solution with using phenol-phthalein as an indicator to determine the terminal carboxyl group content. This value is hereinafter referred to as number-average molecular weight by end-group determination. While the number-average molecular weight by end-group determination is an absolute value, that by GPC determination is a relative value that varies depending on various analytical conditions (for example, kind of the mobile phase, kind of the column, reference substance, selection of slice width, selection of baseline, etc.). It is therefore difficult to have an absolute numerical representation of the latter. However, for example, in the case of a polymer having a terminal carboxyl group and produced from lactic acid and glycolic acid by catalyst-free polycondensation, the number-average molecular weight by GPC and the number-average molecular weight by end-group determination almost agree with each other. The description that the number-average molecular weight by GPC and end-group determination "almost agree" here denotes that the latter falls within the range from about 0.2 to about 1.5 times, preferably about 0.3 to about 1.2 times of the former.

The lactic acid-glycolic acid polymer can be produced by, for example, catalyst-free polycondensation from lactic acid and glycolic acid (JP 61-28521 A) or ring-opening polymerization with catalyst from cyclic lactide, glycolide, etc. (Encyclopedic Handbook of Biomaterials and Bioengineering Part A: Materials, Volume 2, Marcel Dekker, Inc. (1995)).

The polymer produced by ring-opening polymerization has little or no carboxyl group, however, a polymer having a terminal carboxyl group obtained by chemically treating the former polymer (J. Controlled Release, Vol. 41, pages 249-257 (1996)) can be used for the present invention.

The above-mentioned lactic acid-glycolic acid polymer having a terminal carboxyl group can be synthesized by general synthetic methods (e.g. catalyst-free polycondensation described in JP 61-28521 A), without any problem. Moreover, the polymer having free carboxyl groups at unspecified position can be synthesized by known methods (for example, WO94/15587).

As the lactic acid-glycolic acid polymer having a terminal carboxyl group, obtained by chemical treatment after ring-opening polymerization, that available from, for example, Boehringer Ingelheim KG can be employed. These biodegradable polymers can be used alone or in combination with two or more kinds of the polymers.

The polyvalent metal to be used in the present invention may be any metal, as long as it is a metal that does not adversely affect the living body. Examples of the metal include polyvalent metals such as a divalent metal (e.g., iron, zinc, copper, calcium, magnesium, aluminum, tin, manganese, etc.), a trivalent metal (e.g., iron, aluminum, manganese, etc.), a tetravalent metal (e.g., tin, etc.), etc.

The "polyvalent metal compound" to be used in the present invention may be used in the form of a compound with an inorganic or organic substance, a metal oxide, or a metal ion, while a component obtained by treating a metal oxide with water is preferably used.

Preferred examples of the polyvalent metal include iron, aluminum, zinc, calcium, magnesium and the like and an especially preferred example is zinc.

Examples of the inorganic substance to be used include a hydrogen halide (e.g., hydrochloric acid, hydrobromic acid, hydrogen iodide, hydrofluoric acid, etc.), sulfuric acid, nitric acid, thiocyanic acid, and the like.

Examples of the organic substance to be used include organic acids such as aliphatic carboxylic acids, aromatic acids, etc., acethylacetone, and the like. The aliphatic carboxylic acids are preferably aliphatic carboxylic acids having 1 to 9 carbon atoms (e.g., aliphatic monocarboxylic acids, aliphatic dicarboxylic acids, aliphatic tricarboxylic acids, etc.). The aliphatic carboxylic acids may be either saturated or unsaturated.

As the aliphatic monocarboxylic acids, for example, there can be used saturated aliphatic monocarboxylic acids having 1 to 9 carbon atoms (e.g., carbonic acid, acetic acid, propionic acid, butylic acid, valeric acid, caproic acid, enanthic acid, caprylic acid, pelargonic acid, capric acid, etc.), aliphatic unsaturated monocarboxylic acids having 2 to 9 carbon atoms (e.g., acrylic acid, propiolic acid, methacrylic acid, crotonic acid, isocrotonic acid, etc.), and the like.

As the aliphatic dicarboxylic acids, for example, there can be used saturated aliphatic dicarboxylic acids having 2 to 9 carbon atoms (e.g., malonic acid, succinic acid, glutaric acid, adipic acid, pimelic acid, etc.), aliphatic unsaturated dicarboxylic acids having 2 to 9 carbon atoms (e.g., maleic acid, fumaric acid, citraconic acid, mesaconic acid, etc.), and the like.

As the aliphatic tricarboxylic acids, for example, there can be used saturated aliphatic tricarboxylic acids having 2 to 9 carbon atoms (e.g., tricarballylic acid, 1,2,3-butanetricarboxylic acid, etc.), and the like.

The above aliphatic carboxylic acid may have one or two hydroxyl groups and examples of such acid include glycolic acid, lactic acid, glyceric acid, tartronic acid, malic acid, tartaric acid, citric acid, and the like.

The aliphatic carboxylic acid is preferably the aliphatic monocarboxylic acid and more preferably the aliphatic monocarboxylic acid having 2 to 9 carbon atoms. A particularly preferred example of the aliphatic carboxylic acid is acetic acid.

As the aromatic acids, for example, there can be used benzoic acid, salicylic acid, phenolsulfonic acid, and the like.

Examples of the metal compounds include:
salts formed by iron and inorganic acids [e.g., iron halides (such as iron chloride, iron bromide, iron iodide, iron fluoride, etc.), iron sulfate, iron nitrate, iron thiocyanate, etc.], salts formed by iron and organic acids [e.g. iron aliphatic carboxylate (such as iron carbonate, iron acetate, iron glycolate, iron lactate, iron tartarate, etc.), aromatic iron salts (such as iron benzoate, iron salicylate, iron phenolsulfonate, etc.)], iron acetylacetonate, and the like;
salts formed by zinc and inorganic acids [e.g. zinc halides (such as zinc chloride, zinc bromide, zinc iodide, zinc fluoride, etc.), zinc sulfate, zinc nitrate, zinc thiocyanate, etc.], salts formed by zinc and organic acids [e.g. zinc aliphatic carboxylate (such as zinc carbonate, zinc acetate, zinc glycolate, zinc lactate, zinc tartarate, etc.), aromatic zinc salts (such as zinc benzoate, zinc salicylate, zinc phenolsulfonate, etc.)], zinc acetylacetonate, and the like;
salts formed by calcium and inorganic acids [e.g. calcium halides (such as calcium chloride, calcium bromide, calcium iodide, calcium fluoride, etc.), calcium sulfate, calcium nitrate, calcium thiocyanate, etc.], salts formed by calcium and organic acids [e.g. calcium aliphatic carboxylate (such as calcium carbonate, calcium acetate, calcium propionate, calcium oxalate, calcium tartarate, calcium lactate, calcium citrate, calcium gluconate, etc.), aromatic calcium salts (such as calcium benzoate, calcium salicylate, etc.)], calcium acetylacetonate, and the like;
salts formed by magnesium and inorganic acids [e.g. magnesium halides (such as magnesium chloride, magnesium bromide, magnesium iodide, magnesium fluoride, etc.), magnesium sulfate, magnesium nitrate, magnesium thiocyanate, etc.], salts formed by magnesium and organic acids [e.g. magnesium aliphatic carboxylate (such as magnesium carbonate, magnesium acetate, magnesium propionate, magnesium oxalate, magnesium tartarate, magnesium lactate, magnesium citrate, magnesium gluconate, etc.), aromatic magnesium salts (such as magnesium benzoate, magnesium salicylate, etc.)], magnesium acetylacetonate, and the like; and
metal oxides (e.g. zinc oxide, iron oxide, calcium oxide, magnesium oxide, aluminum oxide, copper oxide, manganese oxide, etc.).

As the polyvalent metal, zinc oxide, iron chloride, iron acetylacetonate, zinc acetate, zinc acetylacetonate, calcium acetate, calcium acetylacetonate, magnesium acetate, magnesium acetylacetonate, etc. are preferred, with zinc oxide being more preferred.

In the "component obtained by treating with water a polyvalent metal compound slightly soluble in water" of the present invention, a polyvalent metal (a polyvalent metal compound untreated with water), which is the same as or different from the polyvalent metal used as a starting material, may co-exist in the sustained-release preparation of the present invention, and such a polyvalent metal may exist in the form of a polyvalent metal compound (a compound with inorganic substances or organic substances, or in form of metal oxides, etc.), or in the form of a metal ion. Further, it may form a complex with either one or both of a physiologically active compound, its prodrug or their salt and a biodegradable polymer.

In the present invention, a part of the polyvalent metal which may be incorporated into the preparation may be in the form of a salt of a biodegradable polymer with zinc or a different kind of metals. Such a salt of the biodegradable polymer can be produced by the process described in, for example, JP 9-221420 A or a process similar thereto.

Preferred examples of a process for producing the preparation of the present invention include a process comprising removing water' and a solvent from a solution containing a physiologically active compound slightly soluble in water, a component obtained by treating with water a polyvalent metal compound slightly soluble in water, and a biodegradable polymer, and the like. A polyvalent metal other than the polyvalent metal derived from the "component obtained by treating with water a polyvalent metal compound slightly soluble in water" may be incorporated into the solution by using a complex of the polyvalent metal with either or both of the physiologically active compound and the biodegradable polymer as a starting material. Alternatively, a polyvalent metal other than the polyvalent metal derived from the "component obtained by treating with water a polyvalent metal compound slightly soluble in water" may be incorporated into the solution by adding the other polyvalent metal to water to be used for treatment of the polyvalent metal compound when preparing the "component obtained by treating with water a polyvalent metal compound slightly soluble in water". A part or all of the polyvalent metal other than the polyvalent metal derived from the "component obtained by treating with water the polyvalent metal compound slightly soluble in water" may form a complex with either one or both of the physiologically active compound and the biodegradable polymer.

The amounts of the physiologically active compound and the "component obtained by treating with water the polyvalent metal compound slightly soluble in water" vary depending on a particular type of the physiologically active compound, the desired pharmacological efficacy, the durability of the efficacy, and the like. Normally, in case of using the physiologically active compound, the "component obtained by treating with water a polyvalent metal compound slightly soluble in water", and the biodegradable polymer as starting materials, the physiologically active compound is about 1 to about 5% by weight, preferably about 15 to about 45% by weight, and especially preferably about 20 to about 40% by weight based on the total of these three components, while the "component obtained by treating with water the polyvalent metal compound slightly soluble in water" is about 0.5 to about 20% by weight, preferably about 1 to about 15% by weight, and especially preferably about 2 to about 10% by weight based on the total of these three components.

The sustained-release preparation of the present invention may be administered in any form and is preferably formulations for non-oral administration. Examples of said formulations include percutaneous formulations, indwellable formulations, injectable microcapsules, etc. Among them, injectable microcapsules are preferable, since they are long in duration for maintaining pharmaceutical effect and reduce a burden on the patient.

### Best Modes for Carrying Out the Invention

The process for producing the sustained-release preparation of the present invention, e.g. microcapsule (hereinafter also referred to as microsphere), which comprises the physiologically active compound, a component produced from zinc oxide by short-time treatment and a biodegradable polymer is illustrated hereinafter.

### (I) in-water drying

A physiologically active compound slightly soluble in water, one or more kinds of polyvalent metal compounds slightly soluble in water, and water are added to a solution of a biodegradable polymer in an organic solvent so that the amounts of respective components are within the above-described ranges to obtain a solution of the biodegradable polymer in an organic solvent which contains water, the physiologically active compound slightly soluble in water and "a component obtained by treating with water a polyvalent metal compound slightly soluble in water". At this time, a part of the physiologically active compound slightly soluble in water, one or more kinds of the polyvalent metal compounds slightly soluble in water, and water may not necessarily be dissolved in a solution of the biodegradable polymer in an organic solvent and may be dispersed therein. It is preferred to be dispersed finely within a short period of time by a known method using a homogenizer or ultrasonication.

Water may be added in the absence of either one or both of the physiologically active compound slightly soluble in water and the biodegradable polymer, in so far as the polyvalent metal compound slightly soluble in water is present and it can be sufficiently mixed by a known method.

For example, this can be considered to one process of a multi-steps for preparing a solution of the biodegradable polymer containing the physiologically active compound, wherein, at first, the polyvalent metal compound slightly soluble in water together with water are added to a solution of the biodegradable polymer in an organic solvent, and the mixture is thoroughly mixed, followed by addition of the physiologically active compound thereto and dispersion again.

Examples of the organic solvent include halogenated hydrocarbons (e.g., dichloromethane, chloroform, dichloroethane, trichloroethane, tetrachlorocarbon. etc.), ethers (e.g., ethyl ether, isopropyl ether, etc.), fatty acid esters (e.g., ethyl acetate, butyl acetate, etc.), aromatic hydrocarbons (e.g., benzene, toluene, xylene, etc.), alcohols (e.g., ethanol, methanol, etc.), acetonitrile, and the like. These solvents may be mixed in an appropriate ratio. Among them, preferred are dichloromethane as a halogenated hydrocarbon, and ethanol and methanol as alcohols. They may be mixed in an appropriate ratio.

To the above-described organic solvent solution, an additive may be added. Examples of the additives include solubilizer which maintains stability and solubility of the drug such as carbonic acid, oxalic acid, citric acid, phosphoric acid, hydrochloric acid, sodium hydroxide, arginine, lysine and their salts, etc. Further, as stabilizers of the drug, there can be added, for example, albumin, gelatin, citric acid, sodium ethylenediaminetetraacetate, dextrin, sodium hydrogensulfite, polyols such as polyethyleneglycol, etc., etc., and as preservatives there can be added, for example, conventional para-oxybenzoic acid esters (e.g., methylparaben, propylparaben, etc.), benzylalcohol, chlorobutanol, thimerosal, etc.

The concentration of the biodegradable polymer in the organic solvent solution varies depending on the molecular weight of the biodegradable polymer and kind of the organic solvent. For example, when dichloromethane is used as an organic solvent, the concentration of the biodegradable polymer in the organic solvent solution is generally selected from the range of about 0.5 to about 70% by weight, more preferably about 1 to about 60% by weight, and in particular about 2 to about 50% by weight.

Further, when ethanol or methanol and dichloromethane is used as a mixed organic solvent, the concentration of dichloromethane in the organic solvent solution is generally selected from the range of about 10 to about 99% by volume, more preferably about 20 to about 98% by volume, and in particular about 30 to about 95% by volume.

The thus obtained organic solvent solution of the biodegradable polymer containing the physiologically active compound slightly soluble in water and the "component obtained by treating with water a polyvalent metal compound slightly soluble in water" is added to an aqueous phase to form an O(oil phase)/W(aqueous phase) emulsion, followed by evaporation of water and the solvent in oil phase to yield microcapsules. The volume of the aqueous phase is generally selected from the range of about 1 to about 10,000 times the volume of the oil phase, more preferably about 5 to about 5,000 times, and in particular, about 10 to about 2,000 times.

Any emulsifier may be added to the above outer aqueous phase, as long as it can contribute to the formation of a stable O/W emulsion. Examples of the emulsifiers include anionic surfactants (sodium oleate, sodium stearate, sodium lauryl sulfate, etc.), non-ionic surfactants (polyoxy-ethylene-sorbitan fatty acid esters [Tween 80, Tween 60; Atlas Powder], polyoxyethylene-castor oil derivatives [HCO-60, HCO-50; Nikko Chemicals], etc.), polyvinylpyrrolidone, polyvinylalcohol, carboxymethylcellulose, lecithin, gelatin, hyaluronic acid, etc. These emulsifiers can be used alone or in combination. The concentration may be selected from about 0.01 to about 10% by weight, preferably about 0.05 to about 5% by weight.

To the outer aqueous phase, an osmotic pressure adjustor may be added. Any osmotic pressure adjustor can be employed so long as it produces osmotic pressure in an aqueous solution thereof.

Examples of the osmotic pressure adjustor include polyhydric alcohols, monohydric alcohols, monosaccharides, disaccharides, oligosaccharides, amino acids or their derivatives, etc.

Examples of the above polyhydric alcohols include dihyrdic alcohols such as glycerin, etc., pentahydric alcohols such as arabitol, xylitol, adonitol, etc., hexahydric alcohols such as mannitol, sorbitol, dulcitol, etc., etc. Among others, hexahydric alcohols are preferable and in particular, mannitol is preferable.

Examples of the above monohydric alcohols include methanol, ethanol, isopropyl alcohol, etc. Among others, methanol is preferable.

Examples of the above monosaccharides include pentoses such as arabinose, xylose, ribose, 2-deoxyribose, etc., hexoses such as glucose, fructose, galactose, mannose, sorbose, rhamnose, fucose, etc. Among others, hexoses are preferable.

Examples of the above oligosaccharides include trisaccharides such as maltotriose, raffinose, etc., tetrasaccharides such as stachyose, etc., etc. Among others, trisaccharides are preferable.

Examples of the derivatives of the above mentioned monosaccharides, disaccharides and oligosaccharides include glucosamine, galactosamine, glucuronic acid, galacturonic acid, etc.

Examples of the amino acids include any one of L-isomers and preferred examples include glycine, leucine, arginine, etc. Among others, L-arginine is preferable.

These osmotic pressure adjustors may be used alone or as a mixture of two or more of them.

These osmotic pressure adjustors are usually used at the concentration which makes osmotic pressure of outer aqueous phase about 1/50 to about 5 times, preferably about 1/25 to about 3 times of that of physiological saline.

To remove the organic solvent, per se known methods or methods analogous thereto are employed. For example, it is carried out by evaporating the organic solvent by stirring with a propeller-type stirrer, magnetic stirrer, etc. under atmospheric pressure or gradually reducing pressure or while controlling degree of vacuum by using a rotary evaporator, etc., etc.

The thus obtained microcapsules are collected by centrifugation or filtration. Then, the physiologically active compound, carriers therefor, emulsifiers, etc. attached onto the surface of the microcapsules are washed with distilled water repeatedly several times, dispersed in distilled water, etc., and subjected to freeze-drying. In freeze-drying, aggregation inhibitors may be added to avoid aggregation of the particles during production. Examples of said aggregation inhibitors include water-soluble polysaccharides such as mannitol, lactose, glucose, starch (e.g., corn starch, etc.), etc., amino acid such as glycine, etc., protein such as fibrin, collagen, etc., etc. Among others, mannitol is preferable.

After freeze drying, further removal of water and an organic solvent may be carried out by warming the microcapsules under reduced pressure and under the conditions where the microcapsules do not attach to each other, if desired. Preferably, the microcapsules are warmed at an appropriate temperature which is a little bit higher than median glass transition temperature (determined using differential scanning calorimeter at temperature increments of 10 or 20°C per minute) of the biodegradable polymer. More preferably, the microcapsules are warmed at temperatures ranging from median glass transition temperature to about 30 °C higher than median glass transition temperature of the biodegradable polymer. In particular, when lactic acid-glycolic acid polymer is used as the biodegradable polymer, the microcapsules are preferably warmed at temperatures ranging from median glass transition temperature to about 10°C higher than median glass transition temperature of the polymer, more preferably from median glass transition temperature to about 5°C higher than median glass transition temperature of the polymer.

Warming time varies depending on an amount of the microcapsules to be treated, etc. In general, about 12 hours to about 168 hours, preferably about 24 hours to about 120 hours, and in particular, about 48 hours to about 96 hours after temperature of microcapsules themselves reach to the desired temperature are preferable.

A method for warming the microcapsules is not limited to a specific method, and any method can be employed as long as a set of the microcapsule are uniformly warmed.

Examples of the method for warming and drying the microcapsules include that in constant temperature bath, fluidized bath, moving bath or kiln, that by micro-wave, etc. Among others, the method for warming and drying the microcapsules in constant temperature bath is preferable.

Further, in order to remove water and the organic solvent in the microcapsule, a method using supercritical fluid (CO₂, etc.), pressurized gas (CO₂, etc.) or the like can also be employed.

### (II) phase separation

In producing microcapsules by the phase separation method, an coacervating agent is gradually added to the above-described organic solvent solution of the biodegradable polymer containing water, the physiologocally active compound slightly soluble in water, and the "component obtained by treating with water a polyvalent metal compound slightly soluble in water" as described in the above (I), in-water drying, during stirring to precipitate and solidify the microcapsules. The volume of the coacervating agent is generally selected from the range of about 0.01 to about 1,000 times the volume of the oil phase, more preferably about 0.05 to about 500 times, and in particular, about 0.1 to about 200 times.

Any coacervating agent is acceptable, as long as it is a polymer, mineral oil, vegetable oil, etc. that is miscible in the organic solvent and does not dissolve both of the physiologically active compound and the biodegradable polymer. For example, silicon oil, sesame oil, soybean oil, corn oil, cotton seed oil, coconut oil, linseed oil, mineral oil, n-hexane, n-heptane, etc. are employed. These may be used in combination.

The thus obtained microcapsules are separated, repeatedly washed with heptane, etc. to remove the coacervating agent, other than the physiologically active compound and the biodegradable polymer, and then dried under reduced pressure. Alternatively, the microcapsules are washed with the methods similar to those described in the above (I) in-water drying, subjected to freeze-drying, and then warmed and dried.

### (III) spray drying

In producing microcapsules by this method, the above-mentioned organic solvent solution of the biodegradable polymer containing water, the physiologically active compound slightly soluble in water and the "component obtained by treating with water a polyvalent metal compound slightly soluble in water" as described in the above (I) in-water drying, is sprayed via a nozzle into the drying chamber of a spray drier to volatilize the organic solvent in the fine droplets in a very short time to yield the microcapsule. Examples of the nozzles include double-fluid nozzle, pressure nozzle, rotary disc nozzle, etc. Thereafter, if necessary, the microcapsules are washed with the methods similar to those described in the above (I) in-water drying, subjected to freeze-drying, and then warmed and dried.

In addition to the above-described dosage form of the microcapsules, the organic solvent solution of the biodegradable polymer containing water, the physiologically active compound slightly soluble in water and the "component obtained by treating with water a polyvalent metal compound slightly soluble in water" as described in the above (I) in-water drying, may be subjected to evaporation of the organic solvent and water while controlling degree of vacuum by using a rotary evaporator, etc., and the residue may be crashed with jet mill, etc. to yield fine powders.

The thus obtained fine powders may be washed with the methods similar to those described in the above (I) in-water drying, subjected to freeze-drying, and then warmed, dried, and, if necessary, removal of solvent with supercritical fluid, pressurized gas, etc.

The release of the drug from the thus obtained microcapsules or fine powders can be controlled by degradation rate of the employed biodegradable polymer and kind and/or amount of the "component obtained by treating with water a polyvalent metal compound slightly soluble in water" and water.

The sustained-release preparation of the present invention can be used for the production of various preparations, as it is or as a raw material and administered as injections or implants intramuscularly, subcutaneously, into organs, etc.; as transmucosal preparations into nasalcavity, rectum, uterus, etc.; oral preparations (e.g., capsules (e.g., hard capsules, soft capsules, etc.), solid preparations such as granules, powders, etc., liquid preparations such as syrups, emulsions, suspensions, etc., etc.); etc. Also, the sustained-release preparation of the present invention can be administered using needleless injector.

For example, when the sustained-release preparation according to the present invention are to be processed into injections, it is dispersed together with a dispersing agent (e.g., surfactants such as Tween 80, HCO-60, etc., polysaccharides such as sodium hyaluronate, carboxymethylcellulose, sodium alginate, etc., etc.), a preservative (e.g., methylparaben, propylparaben, etc.), an isotonizing agent (e.g., sodium chloride, mannitol, sorbitol, glucose, proline, etc.), etc. to form an aqueous suspension, or it is dispersed together with vegetable oil such as sesame oil, corn oil, etc. to form an oily suspension, said suspensions being actually used as sustained-release injections.

Particle size of the sustained-release preparation of the present invention is selected from the range satisfying the dispersibility and needle-passability requirements when it is used as suspension. For example, the average diameter ranges from about 0.1 to about 300µm, preferably from about 0.5 to about 150µm, more preferably from about 1 to about 100µm.

In order to prepare a sterile preparation of the sustained-release preparation of the present invention, a method for sterilizing all production steps, a method for sterilizing with γ-rays, a method for adding a preservative, etc. are employed, and there is no limitation to a specific method.

With low toxicity, the sustained-release preparation of the present invention can be used in mammals (e.g., human, bovine, swine, dog, cat, mouse, rat, rabbit, etc.) as a safe medicine, etc.

Varying depending on type, content and dosage form of the physiologically active compound as the active component; duration of release of the physiologically active compound; target disease; subject animal; etc., the dose of the sustained-release preparation of the present invention is within the range of an effective amount of the physiologically active compound. For example, the dose per administration of the active component, the physiologically active compound, is preferably chosen within the range from about 0.01 mg to about 10 mg/kg body weight per adult, more preferably from about 0.05 mg to about 5 mg/kg body weight per adult, when the sustained-release preparation is 1-month preparation.

The dose per administration of the sustained-release preparation of the present invention is preferably chosen within the range from about 0.05 mg to about 50 mg/kg body weight per adult, more preferably from about 0.1 mg to about 30 mg/kg body weight per adult.

Number of administrations can be appropriately selected from once per a few weeks, once per a month, or once per a few months (e.g., 3 months, 4 months, 6 months, etc.), etc., depending on type, content and dosage form of the active component, the physiologically active compound, duration of release of the physiologically active compound, target diseases, subject animals, etc.

Further, the sustained-release preparation of the present invention can advantageously be used for patients who are bedridden, are suffered from dementia, pharynic and/or espophageal diseases, diseases of digestive organs, and disorder of eating and swallowing, and are difficult to be medicated by oral administration such as at the time of surgery, or the like.

In case that the physiologically active compound is a compound having AII antagonistic activity, the compound possesses high safety and therefore acute increase of blood concentration just after administration of it does not cause excess reduction of blood pressure. Thus, the sustained-release preparation of the present invention can be used for the diseases described below and can maintain constant concentration of the drug in blood during day and night. Therefore, the dose and numbers of administration can be reduced, compared with administration of conventional oral preparations. Moreover, the change of concentration of the drug in blood is not remarkable and condition of the patient does not change due to interruption of taking the drug, etc. Therefore, it is expected that the treatment effect of the drug become clearer by administration of the sustained-release preparation of the present invention.

As for the safety, the risk of excess decrease in blood pressure is low as compared with that in case of oral administration as described above in the situation of normal use. Even if excess pressure decrease takes place in case of an incident such as a traffic accident, or the like owing to loss of a large quantity of body fluids, pressure increase can immediately be done by intravenous administration of not only angiotensin II but also a medicine (such as catechol amine preparation) to be used usually in an emergency medical care field and continuous pressure increase is also possible by oral administration of a hypotension curing agent, so that not only acute caring treatment at the time of emergency but also long term treatment can be possible.

Examples of the objective diseases of the compound having angiotensin II antagonistic activity as the physiologically active compound include diseases which are caused or promoted to be caused by vascular contraction, proliferation, and organ disorder manifested through an angiotensin II receptor, existence of angiotensin II or factors induced by the presence of angiotensin II.

Examples of such diseases include systemic diseases such as hypertension, within day precision disorder, heart diseases (e.g., cardiac hypertrophy, acute heart failure, congestive heart failure, chronic heart failure, cardiomyopathy, angina pectoris, myocarditis, arrhythmia, tachycardia, myocardial infarction, etc.), cerebrovascular disorder (e.g., asymptomatic cerebrovascular disorder, transient cerebral ischemia, cerebral apoplexy, cerebrovascular dementia, hypertensive cerebropathy, etc.), cerebral edema, cerebral circulatory disorder, recurrence and sequela of cerebrovascular disorder (neurotic symptom, psychic symptom, subjective symptom, disorder in daily living activities, etc.), ischemic peripheral circulatory disorder, myocardial ischemia, venous insufficiency, progression of cardiac insufficiency after myocardial infarction, diabetes mellitus, diabetic complications (diabetic retinopathy, diabetic nephropathy, diabetic neuropathy, etc.), renal diseases (nephritis, glomerulonephritis, glomerulosclerosis, renal failure, thrombotic vasculopathy, complications of dialysis, organ disorder including nephropathy by radiation damage, etc.) arteriosclerosis including atherosclerosis (aneurysm, coronary arteriosclerosis, cerebral arteriosclerosis, peripheral arteriosclerosis, etc.), vascular hypertrophy, vascular hypertrophy or obliteration and organ failure after intervention (percutaneous transluminal coronary angioplasty, stenting, coronary angioscopy, intravascular ultrasound, douche thrombolytic therapy, etc.), vascular re-obliteration and restenosis after bypass, polycythemia, hypertension, organ failure and vascular hypertrophy after transplantation, rejection after transplantation, ocular diseases (glaucoma, ocular hypertension, etc.), thrombosis, multiple organ failure, endothelial dysfunction, hypertensive tinnitus, other cardiovascular diseases (deep vein thrombosis, obstructive impairment, arteriosclerosis obliteran, obliterative thromboangiitis, transient cerebral circulation disorder, Raynaud's disease, Berger disease, etc.), metabolic and/or nutritional disorder (obesity, hyperlipemia, hypercholesterolemia, diabetes mellitus, impaired glucose tolerance, hyperuricacidemia, hyperkalemia, hypernatremia, etc.), nerve degeneration diseases (Alzheimer's disease, Parkinson's disease, amyotrophic lateral sclerosis, AIDS related cerebral symptom, etc.), central nervous system disorder (cerebral hemorrhage, cerebral infarct, their sequela, complication, head injury, spinal injury, cerebral edema, sensory malfunction, sensory functional disorder, autonomic nervous system disorder, autonomic nervous system malfunction, multiple sclerosis, etc.), dementia, defects of memory, disorder of consciousness, amnesia, anxiety symptom, catatonic symptom, disconform mental state, psychopathy, (depression, epilepsy, alcoholism, etc.), inflammatory diseases (diabetic complication such as retinopathy, nephropathy, neuropathy great vessel dysfunction; arthritis such as rheumatoid arthritis, osteoarthritis, rheumatoid myelitis, periostitis; inflammation after operation and injury; remission of swelling; pharyngitis; cystitis; pneumonia; atopic dermatitis; inflammatory intestinal diseases such as Crohn's disease, ulcerative colitis; meningitis, inflammatory ocular disease; inflammatory pulmonary diseases such as pneumonia, pulmonary silicosis, pulmonary sarcoidosis, pulmonary tuberculosis, etc.), allergic diseases (allergic coryza, conjunctivitis, gastrointestinal allergy, pollinosis, anaphylaxis, etc.), chronic obstructive pulmonary disease, interstitial pneumonia, pneumocytis carinni pneumonia, collagen diseases (e.g., systemic lupus erythematosus, scleroderma, polyarteritis, etc.), hepatic diseases (chronic hepatitis, hepatic cirrhosis, etc.), portal hypertension, digestive system disorder (gastritis, gastric ulcer, stomach cancer, stomach disorder after operation, dyspepsia, esophageal ulcer, pancreatitis, colon polyp, cholelithiasis, hemorrhoidal disease, etc.), blood and/or myelopoietic diseases (erythrocytosis, vascular purpura, autoimmune hemolytic anemia, disseminated intravascular coagulation, multiple myelopathy, etc.), bone diseases (fracture, refracture, osteoporosis, osteomalacia, bone Behcet's disease, sclerosing myelitis, rheumatoid arthritis, osteoarthrosis of the knee and joint tissue distruction owing to similar diseases and disorder, etc.), solid ulcer, ulcer [malignant melanoma, malignant lymphoma, cancer of digestive organs (e.g., cancer of stomach, intestine, etc.)], cancer and cachexia following cencer, metastasis cancer, endocrinopathy (Addison's disease, Cushing's syndrome, pheochromocytoma, primary aldosteronism and the like), Creutzfeldt-Jakob disease, urinary organ and/or male genital diseases (cystitis, prostatic hypertrophy, prostatic cancer, sex infectious disease, etc.), female disorder (climacteric disorder, gestosis, endometriosis, hysteromyoma, ovarian disease, breast disease, sex infectious disease, etc.), diseases and disorder related to environment and occupational factors (radiation hazard, hazard by ultraviolet, infrared, or laser beam, altitude sickness, etc.), respiratory diseases (cold syndrome, pneumonia, asthma, pulmonary hypertension, pulmonary thromboembolism, pulmonary thrombosis, etc.), infectious diseases (viral infectious disease by cytomegalovirus, influenza virus, herpes virus, etc., rikettsiosis, bacterial infectious diseases, etc.), toxemia (sepsis, sepsis shock, endotoxin shock, Gram-negative supsis, toxin shock syndrome, etc.), rhinolarygological diseases (Meniere's syndrome, tinnitus, dysgeusia, vertigo, disequilibrium, dysphagia, etc.), skin diseases (keloid, hemangioma, psoriasis, etc.), intradialytic hypotension, myasthenia gravis, chronic skin syndrome, and the like.

In case the physiologically active compound is a compound having angiotensin II antagonistic activity, by suppressing the action of angiotensin II for a long term, disorder and abnormality of biological and physiological functions which cause a variety of diseases accompanying adult disorder and aging can be improved, or accentuation of such disorder and abnormality can be controlled, thereby achieving primary and secondary prophylactic effects or controlling development of disease conditions caused by those diseases and symptoms. Such disorder and abnormality of biological and physiological functions include disorder and abnormality of automatic controlling capability of cerebral circulation and/or renal circulation, disorder of circulation (peripheral, cerebral, microcirculation and the like), disorder of blood-brain-barrier, insulin susceptibility deterioration, salt susceptibility deterioration, abnormal state of coagulation and fibrinolysis system, abnormal state of blood and blood cell components (accentuation of platelet agglutinability, abnormality of erythrocyte transformation, accentuation of leukocyte agglutininig function, rise of blood viscosity, etc.), production and function accentuation of growth factor and cytokine (PDGF, VEGF, FGF, interleukin, TNF-α, MCP-1, etc.), accentuation of proliferation and infiltration of inflammatory cells, accentuation of production of free radical, liposteatosis accentuation, endothelial function disorder, endothelial, cell, and organ disorder, cell morphogenesis change of edema, smooth muscle, and the like (morphogenesis to proliferation type), accentuation of production and function of vasoactive substance and thrombosis inducers (endothelin, thromboxane A2, etc.), abnormal vasoconstriction, impaired glucose tolerance, metabolic disorder (serum lipid disorder, blood sugar disorder, etc.), abnormal cell propagation, vascular rebirth (including abnormal vasa vasorum formation in adventitial coat of atherosclerosis, abnormal capillary reticular formation), and the like. Among them, the compound can be advantageously used as a primary and secondary prophylactic drug for organ disorder accompanied with a variety of diseases (e.g. cerebrovascular disorder and organ disorder following the cerebrovascular disorder, organ disorder following cardiovascular diseases, organ disorder following diabetes mellitus, organ disorder after intervention).

In the sustained-release preparation of the present invention, when the physiologically active compound is a compound having angiotensin II antagonistic activity (especially, candesartan, cilexetil, candesartan, etc.), it can be advantageously used as a prophylactic and therapeutic drug for portal hypertension. It is well known that esophagovaritosis occurs frequently during night (Hepatology 1994; 19: 595-601), and since the preparation of the present invention is capable of maintaining a constant blood level all day long, the preparation of the present invention is not only capable of decreasing the dosage and the number of administration as compared with those in case of a preparation for oral administration, but also expected to realize stable lowering of portal vein pressure owing to the slight fluctuation of the blood level of the drug. The above characteristics of the preparation show usefulness as a preventive drug for rupture of varicose vein in esophagus and stomach. Further, since no symptom change is caused owing to interruption of the agent administration, it is also expected to clarify the therapeutic effect. Further, a compound having angiotensin II antagonistic activity as the physiologically active compound (especially, candesartan cilexetil, candesartan) is expected to be efficacious on the production and promotion of HGF (hepatocyte growth factor) and to attribute to liver regeneration and liver function restitution.

Further, it is expected to be able to further clarify the prophylactic and therapeutic efficacy on the cerebrovascular disorder such as cerebral infarct, etc. by maintaining a constant blood level of a compound having angiotensin II antagonistic activity as the physiologically active compound (especially, candesartan cilexetil, candesartan) all day long.

In case the physiologically active compound is a compound having angiotensin II antagonistic activity, as a method for treating a patient, it can also be thought that a preparation for oral administration of the angiotensin II antagonist is firstly administered to the patient for a certain period to confirm the reaction of the patient, and then the sustained-release preparation of the present invention is administered. The angiotensin II antagonist to be used for the preparation for the oral administration may be the same as or different from the angiotensin II antagonist contained in the sustained-release preparation. In addition, a hypotensive other than the angiotensin II antagonist (e.g. calcium antagonist, diuretic, β-blocker, etc.) is firstly administered orally to confirm the reaction of a patient, and then the sustained-release preparation of the present invention can be administered. Further, a diuretic hypotensive drug (a preparation for oral administration) which is commonly used in combination with the angiotensin II antagonist may be used in combination with the sustained-release preparation of the present invention.

Further, the sustained-release preparation may be used in combination with other medical components including lipid decreasing drug, cholesterol decreasing drug, HMG-Co, 3-hydroxy-3-methylglutaryl coenzyme A reductase, insulin sensitivity improving drug, bone disease therapeutic drug, myocardial protection drug, coronary arterial disease therapeutic drug, other hypertension therapeutic drug, chronic heart failure therapeutic drug, diabetic mellitus therapeutic drug, liver disease therapeutic drug, intestine and/or duodenal ulcer therapeutic drug, biliary disorder therapeutic drug, dysthyroidism therapeutic drug, nephrosis syndrome therapeutic drug, chronic renal failure therapeutic drug, female disease therapeutic drug, urinary organs and/or male genital disease therapeutic drug, and infection therapeutic drug. In this case, these compounds may be administered in the form of an oral preparation and in some cases, in the form of a rectal preparation or suppository. In this case, examples of components which can be used in combination include fibrates (e.g., clofibrate, benzafibrate, GM fibrozyl, etc.), nicotinic acid and its derivatives and analogues (e.g., acipimox, probucol), bile acid-conjugated resin (e.g. cholestyramine, questinol, etc.), compounds suppressing cholesterol absorption (e.g., sitosterol, neomycin, etc.) squalene epoxidase inhibitor (e.g., NB-598 and analogous compounds, etc.), and the like.

Examples of other components which can be used in combination include oxidosqualene-lanosterolcyclase such as decaline derivatives, azadecaline derivatives, and indane derivatives.

Furthermore, combinations with the following various therapeutic drugs are also possible.

Hypertension therapeutic drugs: diuretic [e.g., furosemide (Lasix), bemetanid (Lunetoron), azosemido (Diart)]; hypotensive drug [e.g., ACE inhibitor (enalapril maleate (Renivace), etc.), Ca antagonist (manidipine, amlodipine), α- or β-receptor inhibitor, etc.] and the like;

Chronic heart failure therapeutic drugs: cardiotonic [e.g., cardiotonic glycoside (Digoxin, etc.), β-receptor stimulus agent (catecholamin preparation such as denopamine and dobutamine), PDE inhibitor. etc.], diuretic agent [e.g., furosemide (Lasix), spironolactone (Aldacton), etc.], ACE inhibitor [e.g. enalapril maleate (Renivace), etc.], Ca antagonist (e.g., amlodipine), β-receptor inhibitor, and the like;

Antiarryhthmic drugs: disopyramide, lidocaine, quinidine sulfate, flecainide acetate, mexiletine hydrochloride, amiodarone, β-blocker, Ca antagonist, and the like;

Bone disease therapeutic drugs: calcium preparation (e.g. calcium carbonate, etc.), calcitonine preparation, activated type vitamin D₃ preparation [e.g., alfacalcidol (Alfarol), calcitriol (Rocaltrol), etc.], sex hormones (e.g., estrogen, estradiol, etc.), hormone preparation [e.g., estrogen conjugated (Premarin), etc.], ipriflavone preparation (Osten, etc.), vitamin K₂, vitamin K₂ preparation [e.g., menatetrenone (Glakay), etc.], bisphosphonate preparation (etidronate, etc.), prostaglandin E2, fluorine compound (e.g., sodium fluoride, etc.), bone matrix protein (BMP), fibroblast growth Factor (FGF), platelet-derived growth factor (PDGF), transforming growth factor (TGF-β), insulin-like growth factor 1 and 2 (IGF-1, -2), parathyroid hormone (PTH), compounds disclosed in EP-A1-376197, EP-A1-460488, and EP-A1-719782 [e.g., (2R,4S)-(-)-N-[4-(diethoxyphosphorylmethyl)phenyl]-1,2,4,5-tetrahydro-4-methyl-7, 8-methylenedioxy-5-oxo-3-benzothiepin-2-carboxyamide, etc.], and the like;

Diabetes mellitus therapeutic drugs; Actos, rosiglitazone, Kinedak, Penfill, Humulin, Euglucon, Glimicron, Daonil, Novolin, Monotard, insulins, Glucobay, Dimelin, Rastinon, versylcone, Deamelin-S, Iszilin, and the like;

Liver disease therapeutic drugs: glycyrrhizin preparation (e.g., Strong Minophagen, etc.), liver lysosome, sulfhydryl compound (e.g., glutathione, etc.), special amino acid preparation (e.g., Aminoleban, etc.), phospholipid (e.g., polyenephosphatidyl choline, etc.), vitamins (e.g., vitamin B₁, B₂, B₆, B₁₂, C, etc.), adrenocortical hormone (e.g., dexamethasone, betamethasone, etc.), interferon (e.g., interferon α, β, etc.), hepatic cerebropathy therapeutic drug (e.g., lactulose, etc.), hemostatic to be employed in esophagus and/or gastric varicosis rupture (e.g., vasopressin, somatostatin, etc.), and the like;

Gastric and/or duodenal ulcer therapeutic drugs: antiacid [e.g., histamine H2 receptor antagonists (cimetidine, etc.), proton pump inhibitor (lansoprazole, etc.), and the like;

Biliary tract therapeutic drugs: choleretic (e.g., dehydrocholic acid, etc.), cholekinetic (e.g., magnesium sulfate, etc.), and the like; Hypothyroidism therapeutic drugs: dry thyroidea (Thyreoid), levothyroxine sodium (Thyradin-S), liothyronine sodium (Thyronin, Thyronamin), and the like;

Nephritic syndrome therapeutic drugs: for steroid therapy which is normally employed as primarily selection, prednisolon (Predonine), prednisolone sodium succinate (Predonine), methylprednisolone sodium succinate Solumedrol), betamethasone (Rinderone), and the like are used; and for anticoagulation therapy, dipyridamole (Persantin), dilazep dihydrochloride (Comelian), teclopidine, clopidogrel, antiplatelet drug such as Fxa inhibitor, etc. and anticoagulant are used;

HMG-CoA reductase inhibitors: cerivastatin, atorvastatin, provastatin, simvastatin, itavastatin, lovastatin, fluvastatin, (+)-3R,5S-7-(4-(4-fluorophenyl)-6-isopropyl-2-(N-methyl-N-methanesulfonylamino)pyridin-5-yl)-3,5-dihydroxy-6(E)-heptenoic acid, and the like;

Chronic renal failure therapeutic drugs: usable in combination with diuretic agent [e.g., furosemide (Lasix), bumetanid (Lunetoron), azosemido (Diart)], hypotensive drug [e.g., ACE inhibitor, enalapril maleate (Renivace)], calcium antagonist (manidipine) and α-adrenergic receptor antagonist, preferably by oral administration at the time of administration;

Thrombosis prophylactic drugs: anticoagulant [e.g., heparin sodium, heparin calcium, warfarin potassium (warfarin), blood coagulation factor Xa inhibitor and drugs capable of correcting balancing function of coagulation system], thromgolytic (e.g., tPA, urokinase), antiplatelet [e.g., aspirin, sulfinpyrazon (Anturan), dipyridamole (Persantin), ticropidine (Panaldine), cilostazol (Pletaal), GPIIb/IIIa antagonist (ReoPro)], and the like;

Coronary vasodilators: nifedipine, diltiazem, nicorandil, nitrite agent, and the like;

Cardioplegia drugs: opening drug for heart ATP-K, Na-H antiporters inhibitor, endothelin antagonist, urotensin antagonist, and the like;

Anti-inflammatory drugs: aspirin, acetaminophen, nonsteroidal anti-inflammatory drug (e.g., indometacine, etc.), steroid agent (e.g., dexamethasone, etc.), and the like;

Antiallergic drugs: antihistamine drug (e.g., chloropheniramine maleate, etc.), drug for stimulus therapy (e.g., bucillamine, etc.), azelastine hydrochloride, seratrodast, tranilast, oxatomide, Stronger Neo Minophagen C, tranexamic acid, ketotifen fumarate, and the like;

Antineoplastic drugs: alkylating agent, metabolic antagonist, antitumor antibiotic preparation, antitumor plant-derived component preparation, and the like,

Central nervous system active drugs: anxiolytic, sleep sedative, anesthetic, antispasmodic, autonomic drug, drug for Parkinson's disease, drug for psychoneurosis, and the like;

Therapeutic drugs for female diseases: [e.g., therapeutic drug for climacteric disorder (estrogen conjugated, estradiol, testosterone enanthate, estradiol valerate, etc.), breast cancer therapeutic drug (tamoxifen citrate, etc.), endometriosis and/or myeoma uteri therapeutic drug (leuprorelin acetate, danazol, etc.)], and the like;

Urinary organ and/or male genital disease therapeutic drugs: [e.g., prostatic hypertrophy (tamsulosin hydrochloride, prazosin hydrochloride, chlormadinone acetate, etc.), prostatic cancer (leuprorelin acetate, goserelin acetate, chlormadinone acetate, etc.)], and the like;

Infectious disease therapeutic drugs: [e.g., antibiotic preparation (cefotiam dihydrochloride, cefozopran hydrochloride, ampicillin, etc.), chemotherapeutic drug (sulfonamido agent, synthetic antibacterial agent, antiviral substance, etc.), biological preparation (vaccine, blood derivatives such as immunogloblin, etc.), and the like]; and others.

In addition, there are antiobesic drugs (Mag-indo-l), antirheumatic, and the like; and, further, there are medical care by introduction of living body-derived various factors and their gene (e.g. ischemic disease therapeutic by vascular regeneration promoting factors such as HGF, VEGF and their gene introduction, etc.), and the like.

In case of using the sustained-release preparation of the present invention in combination with these medical components, respective drugs may be incorporated into the single sustained-release preparation. Alternatively, the above-described medical components can be compounded with pharmacologically acceptable carriers, fillers, binders, diluent and the like to prepare respective preparations, and they are separately or simultaneously administered in combination with the sustained-release preparation of the present invention. In case preparations of these medical components are separately produced, the respective preparations can be mixed using a diluent upon administration, but the respective separate preparations can also be administered to a patient simultaneously or at certain time intervals.

### Examples

Hereinafter, the present invention will be illustrated more in details with the reference to Examples and Experiments, but the present invention is not limited to these Examples and Experiments.

### Reference Example 1

To a solution obtained by dissolving 3.6 g of lactic acid-glycolic acid copolymer (lactic acid/glycolic acid 75/25% by mole, the weight average molecular weight 14,000, the number average molecular weight 4,200, the number average molecular weight determined by terminal group quantitative determination 4,090, produced by Wako Pure Chemical Industries Ltd.) in 11 ml dichloromethane and 0.4 ml ethanol were added 2 g of 2-ethoxy-1-[[2'-(1H-tetrazol-5-yl)biphenyl-4-yl]methyl]benzimidazole-7-carboxylic acid (hereinafter, abbreviated as compound A) and 0.36 g of zinc oxide (Type V, Wako Pure Chemical Industries Ltd.) and the mixture was shaken and stirred at a room temperature for 14 hours to obtain a cloudy solution. The obtained solution was poured to 800 ml of an aqueous solution of 0.1% by weight of polyvinyl alcohol previously adjusted to 15°C, followed by mixing with a turbine type homomixer at 8,500 rpm to obtain an O/W emulsion. The O/W emulsion was stirred at a room temperature for 3 hours to evaporate dichloromethane and ethanol, and solidify the oil phase which was collected with a centrifuge. The oil phase was again dispersed in distilled water and subjected to centrifugation to wash out the free drug, etc. Collected microcapsules were again mixed with distilled water, in which a little mannitol was dissolved, and dispersed and then freeze-dried to obtain the microcapsules in the form of a powder. The entrapment ratio of the compound A in the microcapsules was 98% and the content of the compound A in the microcapsules was 33.0%.

### Comparative Example 1

Microcapsules were obtained according to the same manner as Reference Example 1, except that the 14-hour shaking and stirring was changed to 1-minute dispersing and mixing with a homogenizer. The entrapment ratio of the compound A in the microcapsules was 100% and the content of the compound A in the microcapsules was 33.5%.

### Example 1

Microcapsules were obtained according to the same manner as Comparative Example 1, except that 0.4 ml of distilled water was added and dispersing (emulsifying) and mixing with solid body (the compound A and zinc oxide) was carried out at the same rotation speed for 1 minute with a homogenizer. The entrapment ratio of the compound A in the microcapsules was 97% and the content of the compound A in the microcapsules was 32.6%.

### Example 2

Microcapsules were obtained according to the same manner as Example 1, except that the amount of the added distilled water was changed to 0.08 ml. The entrapment ratio of the compound A in the microcapsules was 97% and the content of the compound A in the microcapsules was 32.5%.

### Experiment Example 1

Each 25 mg of the microcapsules obtained by Reference Example 1, Comparative Example 1, and Examples 1 and 2 were dispersed in a dispersant (a solution obtained by dissolving 5 mg of sodium carboxymethylcellulose, 1 mg of Polysolvate 80, and 50 mg of mannitol in distilled water) and injected to 7-week old male SD rats subcutaneously at the shoulder neck part using 23G injection needle. After administration, with the lapse of time, the rats were sacrificed by bleeding out of abdominal aorta and the microcapsules remaining in the administration site and the compounds in the microcapsules were quantitated and the residual ratios of the compounds were calculated. The results are shown in Table 1.

**Table 1**

| Average remaining ratio of compound after subcutaneous administration of microcapsules (n = 3 to 5) | | | | | |
|---|---|---|---|---|---|
| | one day after | one week after | two weeks after | three weeks after | four weeks after |
| Reference Example 1 | 93% | 76% | 49% | 20% | 6% |
| Comparative Example 1 | 10% | ND | ND | ND | ND |
| Example 1 | 91% | 68% | 17% | 2% | 0% |
| Example 2 | 95% | 76% | 48% | 21% | 3% |
| ND : not done | | | | | |

Release at the initial stage of the physiologically active compound of the preparations of Example 1 and Example 2 containing the component derived from zinc oxide by short time treatment with water significantly was suppressed, like the preparation of Reference Example 1 in which the components including zinc oxide were mixed for a long time to produce interaction between the components, and different from the preparation of Comparative Example 1 which was produced by treating for a short time without adding the water. In case of the preparation produced by short time treatment without adding water of Comparative Example 1, considerably excess release at the initial stage of the physiologically active compound was observed. It was found that the subsequent release rates of the physiologically active compound in case of the preparations of Example 1 and Example 2, in which release at the initial stage was suppressed, were changed depending on the amount of water added during the short time treatment, and that the release rate in the preparation of Example 1 where more amount of water was added was faster than that of the preparation of Example 2 where less water was added, thereby confirming that the release rate of the physiologically active compound could be controlled by the amount of water to be added.

### Example 3

To a solution obtained by dissolving 7.2 g of lactic acid-glycolic acid copolymer (lactic acid/glycolic acid 75/25% by mole, the weight average molecular weight 10,600) in 22 ml dichloromethane and 0.8 ml ethanol were added 4 g of the compound A and 0.72 g of zinc oxide (Type V, Wako Pure Chemical Industries Ltd.), followed by addition of 0.16 ml of distilled water. Immediately after addition of distilled water, the mixture was dispersed (emulsified) and mixed with homogenizer under the same conditions as those in Comparative Example 1 to obtain a cloudy solution. The obtained solution was spread in a circular shape with about 5 cm diameter on a flat plate and dried under reduced pressure at a room temperature for 15 hours to obtain a dried product. The dried product was roughly crushed on a sieve with hole size of 250 µm diameter and 5 g of the dried product passed through the sieve was mixed with 0.4 g of mannitol and pulverized by air at 2 kg/cm² air pressure using a jet mill apparatus (A-OJET manufactured by Seishin Enterprise) to obtain fine particles with an average particle size of 21 µm. The content of the compound A in the particles was 31.0%.

### Example 4

A cloudy solution having the same formulation as, and obtained by dispersing (emulsifying) and mixing according to the same manner as those in Example 3 was spray-dried (Mobile Minor, manufactured by Niro Japan) under the following conditions and treated with cyclone separator to obtain fine particles with an average particle size of 32 µm as a dried product.
spraying manner: binary nozzle (nozzle diameter 1.2 mm)
air pressure: 1 kg/cm²
drying chamber inlet temperature: 90°C
drying chamber outlet temperature: 40-43°C.
The content of the compound A in the fine particles was 28.1%.

### Experiment Example 2

About 5 mg of each of the microcapsules and fine particles obtained by Reference Example 1, Examples 2-4 was added to a glycine buffer solution (pH 10, 25 mM) containing 1% CTAB in a 17 P vial and, after sealing with a rubber stopper, the mixtures were shaken and stirred at 42°C and 120 rpm. With the lapse of time, each 0.1 ml was taken out through the rubber stopper and, after filtration through a 0.25 µm filter, the concentration of the compound A was measured and release ratio was calculated. The results are shown in Table 2.

**Table 2**

| Average release ratio of compound in in vitro release property test (n = 3) | | | | | |
|---|---|---|---|---|---|
| | one hour after | two hours after | four hours after | six hours after | twenty four hours after |
| Comparative Example 1 | 98.6% | - | - | - | - |
| Example 2 | 19.7% | 27.8% | 43.7% | 56.7% | 94.4% |
| Example 3 | 28.9% | 37.4% | 54.1% | 64.6% | 87.2% |
| Example 4 | 16.2% | 25.2% | 41.7% | 60.8% | 94.8% |

The sample of Comparative Example 1 which showed a high release rate in the in vivo test of Experiment Example 1 was found having a high release rate also in the in vitro test. The release property of Example 3 and Example 4 was found to be approximately the same as the release property of Example 2 in which the sustained release for four weeks in the in vivo test was confirmed, so that it was suggested that the samples of Example 3 and Example 4 might have the sustained-release property lasting for about four weeks in vivo.

### Industrial Applicability

The sustained-release preparation of the present invention is a preparation comprising a component obtained by treating with water the polyvalent metal compound slightly soluble in water, and the production of the preparation can be finished within a short period of time. In addition, the preparation contains a high amount of the physiologically active compound and its release rate can be controlled. Then, it is expected to maintain the desired pharmacological activity of the physiologically active compound for a long term.

In the case the physiologically active compound slightly soluble in water is a compound having angiotensin II antagonistic activity, a constant blood level thereof can be maintained and therefore the fluctuation of blood level thereof is less than the case of oral administration. Then, stable and continuous pharmacological activity can be expected. Accordingly, disease conditions hardly take a turn for the worse even in case of a group of patients with slight subjective symptoms who intentionally refuse to take a medicine such as taking at inconstant time, interruption of taking, etc. Thus, more clear therapeutic effect can be expected on not only hypertension, within day precision disorder, heart diseases, (cardiac hypertrophy, heart failure, myocardial infraction. etc.), cerebrovascular disorder (asymptomatic cerebral infarction, transient cerebral ischemia, cerebral apoplexy, cerebrovascular dementia, hypertensive cerebropathy. etc.), ischemic peripheral circulation disorder, arteriosclerosis obliterans, occlusive thromboangiitis, ischemic peripheral circulatory disorder, cardiomyopathy, venous insufficiency, progression of cardiac insufficiency after myocardial infarction, sequelae of cerebrovascular disorder, and the like, but also diabetic complication, diabetic retinopathy, diabetic nephropathy, nephritis, glomerulonephritis, nephropathy owing to radiation, atherosclerosis, arteriosclerosis, vascular hypertrophy, vascular hypertrophy and obliteration after intervention, vascular reobstruction after bypass, polycythemia, hypertension, organ failure, vascular hypertrophy after transplantation, rejection after transplantation, high aldosteronism, glomerulosclerosis, renal failure, portal hypertension, glaucoma, ocular hyperlipemia, hyperlipidemia, angina pectoris, aneurysm, coronary arteriosclerosis, cerebral arteriosclerosis, peripheral arteriosclerosis, thrombosis, central nervous system disorder, Alzheimer's disease, defects of memory, depression, amnesia, senile dementia, sensory disturbance, multiple organ failure, endothelial dysfunction, hypertensive tinnitus, Meniere's syndrome, scleroderma, anxiety following vertigo, tense and uncomfortable psychological state, and further digestion disturbance, autonomic nervous system disorder, myasthenia gravis, cancer and cancer-related diseases, and the like. Further, the sustained-release preparation of the present invention is an excellent medicine which can be used for patients who are bedridden, are suffered from dementia, pharynic and/or espophageal diseases, diseases of digestive organs, and disorder of eating and swallowing, and are difficult to be medicated by oral administration such as at the time of surgery, or the like.

## Claims

1. A sustained-release preparation which comprises a physiologically active compound slightly soluble in water, a component obtained by treating with water a polyvalent metal compound slightly soluble in water, and a biodegradable polymer.

2. The sustained-release preparation according to claim 1, wherein the physiologically active compound is a non-peptide compound.

3. The sustained-release preparation according to claim 1, wherein the physiologically active compound is a compound having angiotensin II antagonistic activity, its prodrug, or a salt thereof.

4. The sustained-release preparation according to claim 3, wherein the compound having angiotensin II antagonistic activity is a non-peptide compound.

5. The sustained-release preparation according to claim 3, wherein the compound having angiotensin II antagonistic activity is a compound containing oxygen atom in its molecule.

6. The sustained-release preparation according to claim 3, wherein the compound having angiotensin II antagonistic activity is a compound having an ether bond or a carbonyl group.

7. The sustained-release preparation according to claim 3, wherein the compound having angiotensin II antagonistic activity is a compound represented by the formula I: 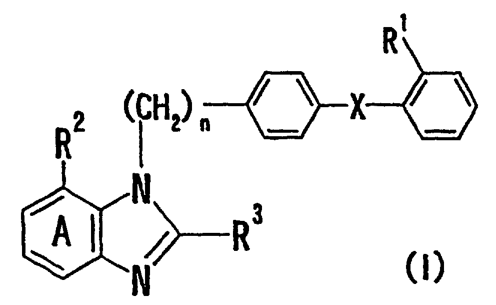 wherein R¹ is a group capable of forming an anion or a group capable of converting thereinto, X shows that the phenylene group and the phenyl group bind to each other directly or through a spacer having an atomic chain length of 2 or less, n is an integer of 1 or 2, the ring A is a benzene ring having an optional substitution, in addition to the group R², R² is a group capable of forming an anion or a group capable of converting thereinto, and R³ is an optionally substituted hydrocarbon residue which may bind through a hetero-atom, or a salt thereof.

8. The sustained-release preparation according to claim 3, wherein the compound having angiotensin II antagonistic activity is Losartan, Eprosartan, Candesartan cilexetil, Candesartan, Valsartan, Telmisartan, Irbesartan, Ormesartan, or Tasosartan.

9. The sustained-release preparation according to claim 3, wherein the compound having angiotensin II antagonistic activity is 2-ethoxy-1-[[2'-(1H-tetrazol-5-yl)biphenyl-4-yl]methyl]benzimidazole-7-carboxylic acid or a salt thereof.

10. The sustained-release preparation according to claim 3, wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate or a salt thereof.

11. The sustained-release preparation according to claim 3, wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid or a salt thereof.

12. The sustained-release preparation according to claim 1, wherein the biodegradable polymer is α-hydroxycarboxylic acid polymer.

13. The sustained-release preparation according to claim 12, wherein the α-hydroxycarboxylic acid polymer is lactic acid-glycolic acid polymer.

14. The sustained-release preparation according to claim 13, wherein the molar ratio of lactic acid and glycolic acid is 100/0-40/60.

15. The sustained-release preparation according to claim 12, wherein the weight-average molecular weight of the polymer is 3,000-50,000.

16. The sustained-release preparation according to in claim 1, which is for injection.

17. The sustained-release preparation according to claim 1, wherein the polyvalent metal is zinc.

18. The sustained-release preparation according to claim 17, wherein the polyvalent metal compound is zinc oxide.

19. The sustained-release preparation according to claim 1 which further comprises a polyvalent metal.

20. The sustained-release preparation according to claim 19, wherein the polyvalent metal is zinc.

21. A process for producing the sustained-release preparation according to claim 1, which comprises removing water and a solvent from a solution containing a physiologically active compound slightly soluble in water, a component obtained by treating with water a polyvalent metal compound slightly soluble in water, and a biodegradable polymer.

22. The process for producing the sustained-release preparation according to claim 21, wherein the physiologically active compound is a compound having angiotensin II antagonistic activity, its prodrug, or a salt thereof.

23. The process for producing the sustained-release preparation according to claim 19, which comprises removing water and a solvent from a solution containing a physiologically active compound slightly soluble in water, a component obtained by treating with water a polyvalent metal compound slightly soluble in water, a biodegradable polymer, and a polyvalent metal.

24. The process for producing the sustained-release preparation according to claim 23, wherein the physiologically active compound is a compound having angiotensin II antagonistic activity, its prodrug, or a salt thereof.

25. A pharmaceutical composition comprising the sustained-release preparation according to claim 1.

26. The composition according to claim 25 for a prophylactic and therapeutic drug for cardiovascular diseases.

27. The composition according to claim 25 for a prophylactic and therapeutic drug for hypertension.

28. The composition according to claim 25 for a prophylactic and therapeutic drug for blood pressure within-day precision abnormality.

29. The composition according to claim 25 for a prophylactic and therapeutic drug for organ disorder.

30. The sustained-release preparation according to claim 1, wherein the component obtained by treating with water a polyvalent metal compound slightly soluble in water is a component obtained by mixing the polyvalent metal compound slightly soluble in water with water.

31. A process for producing a sustained-release preparation of a physiologically active compound slightly soluble in water, which comprises removing water and a solvent from an emulsion obtained by mixing the physiologically active compound slightly soluble in water, a polyvalent metal compound slightly soluble in water and water with a solution of a biodegradable polymer in an organic solvent.

32. A process for producing a sustained-release preparation of a physiologically active compound slightly soluble in water, which comprises dispersing a physiologically active compound slightly soluble in water in an emulsion obtained by mixing a polyvalent metal compound slightly soluble in water and water with a solution of a biodegradable polymer in an organic solvent, and then removing water and a solvent from the dispersion.

33. An emulsion comprising an internal phase containing a physiologically active compound slightly soluble in water, a polyvalent metal compound slightly soluble in water, and water in the internal phase, and an external phase containing a solution of a biodegradable polymer in an organic solvent.

34. A sustained-release preparation obtained by the process according to claim 31.

35. A sustained-release preparation obtained by the process according to claim 32.

36. A method for controlling the release rate of a physiologically active compound slightly soluble in water from a sustained-release preparation, which comprises removing water and a solvent from a solution containing a physiologically active compound slightly soluble in water and a biodegradable polymer in the presence of a compound obtained by treating with water a polyvalent metal compound slightly soluble in water.

37. The method according to claim 35, wherein the amount of water for treatment is adjusted.

38. The method according to claim 35, wherein water and a solvent are removed from an emulsion obtained by mixing a physiologically active compound slightly soluble in water and a polyvalent metal compound slightly soluble in water with a solution of a biodegradable polymer in an organic solvent in the presence of water.

39. The method according to claim 35, wherein a physiologically active compound slightly soluble in water is dispersed in an emulsion obtained by mixing a polyvalent metal compound slightly soluble in water with a solution of a biodegradable polymer in an organic solvent in the presence of water, and water and a solvent are removed from the dispersion.
